(19) Europäisches Patentamt
European Patent Office
Office européen des brevets

(11) EP 1 154 983 B1

(12) EUROPÄISCHE PATENTSCHRIFT

(45) Veröffentlichungstag und Bekanntmachung des Hinweises auf die Patenterteilung:
**28.04.2004 Patentblatt 2004/18**

(21) Anmeldenummer: **00907556.5**

(22) Anmeldetag: **12.02.2000**

(51) Int Cl.[7]: **C07C 217/48**, C07D 295/08, C07D 307/52, C07D 309/14, C07D 233/48, A61K 31/138, A61K 31/4453, A61P 9/06, A61P 25/28

(86) Internationale Anmeldenummer:
**PCT/EP2000/001157**

(87) Internationale Veröffentlichungsnummer:
**WO 2000/048987 (24.08.2000 Gazette 2000/34)**

(54) **NEUE SUBSTITUIERTE 3-PHENOXY- UND 3-PHENYLALKYLOXY-2-PHENYL-PROPYLAMINE**
NOVEL SUBSTITUTED 3-PHENOXY AND 3-PHENYLALKYLOXY-2-PHENYL-PROPYLAMINES
NOUVELLES 3-PHENOXY ET 3-PHENYLALKYLOXY-2-PHENYL-PROPYLAMINES SUBSTITUEES

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **20.02.1999 DE 19907385**

(43) Veröffentlichungstag der Anmeldung:
**21.11.2001 Patentblatt 2001/47**

(73) Patentinhaber: **Boehringer Ingelheim Pharma GmbH & Co.KG**
**55218 Ingelheim am Rhein (DE)**

(72) Erfinder:
- **STRANSKY, Werner**
  **55435 Gau-Algesheim (DE)**
- **GRAUERT, Matthias**
  **55218 Ingelheim am Rhein (DE)**
- **CARTER, Adrian**
  **55411 Bingen am Rhein (DE)**
- **WEISER, Thomas**
  **55268 Nieder-Olm (DE)**
- **BECHTEL, Wolf-Dietrich**
  **55437 Appenheim (DE)**
- **ENSINGER, Helmut**
  **55218 Ingelheim (DE)**
- **LOTZ, Ralf, Richard, Hermann**
  **88433 Schemmerhofen (DE)**
- **PALLUK, Rainer**
  **55411 Bingen am Rhein (DE)**
- **PSCHORN, Uwe**
  **55216 Mainz (DE)**

(56) Entgegenhaltungen:
**EP-A- 0 869 119** **WO-A-94/24116**
**WO-A-97/27169** **DE-A- 2 236 423**

- **JAN HEERES ET AL.: "Antimycotic Imidazoles. 3. Synthesis and Antmycotic Properties of 1-[2-(Aryloxyalkyl)-2-phenylethyl]-1H-imidazoles" JOURNAL OF MEDICINAL CHEMISTRY., Bd. 20, Nr. 11, November 1977 (1977-11), Seiten 1516-1520, XP002139009 AMERICAN CHEMICAL SOCIETY. WASHINGTON., US ISSN: 0022-2623**
- **DATABASE WPI Section Ch, Week 199029 Derwent Publications Ltd., London, GB; Class B03, AN 1990-222320 XP002139010 -& JP 02 152972 A (MORISHITA PHARM CO LTD) , 12. Juni 1990 (1990-06-12)**

Bemerkungen:
Die Akte enthält technische Angaben, die nach dem Eingang der Anmeldung eingereicht wurden und die nicht in dieser Patentschrift enthalten sind.

EP 1 154 983 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Patentanmeldung betrifft neue substituierte 3-Phenoxy- bzw. 3-Phenylalkyloxy-2-phenyl-propylamine der allgemeinen Formel **1**, Verfahren zu ihrer Herstellung und ihre Verwendung als Arzneimittel.

Aminopropanol-Derivate, Verfahren zu ihrer Herstellung und ihre Verwendung in der Humanmedizin aufgrund ihrer Wirkung auf das kariovasküläre System sind aus der DE 2236423 bekannt. 1-[2-(Aryloxyalkyl)-2-phenylethyl]-1*H*-imidazole mit antimykotischen Eigenschaften werden durch Jan Heeres et al., Journal of Med. Chem., 1977, Vol. 20, pages 1516-1520 offenbart. Ferner werden durch die JP 02 152972 A N-Tirazolyl-propanol-Derivate beschrieben. Keine dieser Dokumente offenbart ortho-disubstituierte 3-Phenoxy- und 3-Phenylalkyloxy-2-phenyl-propylamine, die durch eine überlegene biologische Wirksamkeit gekennzeichnet sind.

Die vorliegende Patentanmeldung betrifft solche neuen ortho-disubstituierten 3-Phenoxy- bzw. 3-Phenylalkyloxy-2-phenyl-propylamine der allgemeinen Formel **1**,

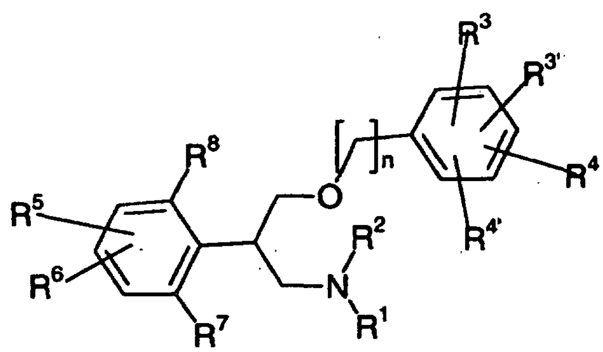

**1**

worin

| | |
|---|---|
| $R^1$ und $R^2$ | unabhängig voneinander Wasserstoff, $C_1$-$C_6$-Alkyl, Benzyl, Furylmethyl, Cycloalkyl, Cycloalkyl-methyl, $C_2$-$C_6$-Alkenyl, bevorzugt Allyl, $C_2$-$C_6$-Alkinyl, bevorzugt, Propargyl, $C_1$-$C_6$-Alkoxy-$(CH_2)_l$-, $C_3$-$C_8$-Cycloalkoxy-$(CH_2)_m$-, und l eine ganze Zahl 1, 2, 3 oder 4, und m eine ganze Zahl 0, 1, 2, 3 oder 4, oder |
| $R^1$ und $R^2$ | zusammen mit dem Stickstoffatom einen 5-, 6- oder 7-gliedrigen heterocyclischen Ring bilden, der gegebenenfalls mit 1-4 Methylgruppen oder einer Dimethylengruppe substituiert sein kann; |
| n | eine ganze Zahl 0, 1, 2 oder 3; |
| $R^3$, $R^4$ und $R^{3'}$ | unabhängig voneinander Wasserstoff, Fluor, Chlor, Brom, Hydroxy, Methyl, Ethyl, Methoxy oder $CF_3$; |
| $R^{4'}$ | Wasserstoff; |
| $R^5$ und $R^6$ | unabhängig voneinander Wasserstoff, Fluor, Chlor, Brom, Methyl oder Ethyl oder |
| $R^5$ und $R^6$ | benachbart einen ankondensierten aromatischen Ring; |
| $R^7$ und $R^8$ | unabhängig von einander Methyl, Ethyl, Methoxy oder Fluor bedeuten können. |

Besonders bevorzugt sind Verbindungen der allgemeinen Formel **1**, worin

| | |
|---|---|
| $R^1$ und $R^2$ | unabhängig voneinander Wasserstoff, $C_1$-$C_6$-Alkyl, Benzyl, Furylmethyl, Cycloalkyl, Cycloalkyl-methyl, $C_2$-$C_6$-Alkenyl, bevorzugt Allyl, $C_2$-$C_6$-Alkinyl, bevorzugt, Propargyl, $C_1$-$C_6$-Alkoxy-$(CH_2)_l$-, $C_3$-$C_8$-Cycloalkoxy-$(CH_2)_m$-, und l eine ganze Zahl 1, 2, 3 oder 4, und m eine ganze Zahl 0, 1, 2, 3 oder 4, oder |
| $R^1$ und $R^2$ | zusammen mit dem Stickstoffatom einen 5-, 6- oder 7-gliedrigen heterocyclischen Ring bilden, der gegebenenfalls mit 1-4 Methylgruppen oder einer Dimethylengruppe substituiert sein kann; |

| | |
|---|---|
| n | eine ganze Zahl 0, 1, 2 oder 3; |
| $R^3$ und $R^4$ | unabhängig voneinander Wasserstoff, Fluor, Chlor, Brom, Hydroxy, Methyl, Ethyl, Methoxy oder $CF_3$; |
| $R^{3'}$ und $R^{4'}$ | Wasserstoff; |
| $R^5$ und $R^6$ | unabhängig voneinander Wasserstoff, Fluor, Chlor, Brom, Methyl, oder Ethyl; |
| $R^7$ und $R^8$ | unabhängig von einander Methyl, Ethyl, Methoxy oder Fluor bedeuten können. |

Erfindungsgemäß von besonderem Interesse sind Verbindungen der allgemeinen Formel **1,** worin:

| | |
|---|---|
| $R^1$ und $R^2$ | unabhängig von einander Wasserstoff, $C_1$-$C_4$-Alkyl, Benzyl, Furylmethyl, Cycloalkyl, Cycloalkyl-methyl, $C_2$-$C_4$-Alkenyl, bevorzugt Allyl, $C_2$-$C_4$-Alkinyl, bevorzugt Propargyl, $C_1$-$C_4$-Alkoxy-$(CH_2)_l$-, $C_3$-$C_6$-Cycloalkoxy-$(CH_2)_m$-<br>und<br>l eine ganze Zahl 1, 2 oder 3,<br>und<br>m eine ganze Zahl 1, 2 oder 3,<br>oder |
| $R^1$ und $R^2$ | zusammen mit dem Stickstoffatom einen 5- oder 6- gliedrigen heterocyclischen Ring bilden, der gegebenenfalls mit 1, 2 oder 3 Methylgruppen oder einer Dimethylengruppe substituiert sein kann; |
| n | eine ganze Zahl 0, 1, 2 oder 3; |
| $R^3$ | Fluor, Chlor oder Methyl, bevorzugt in *ortho*-Position; |
| $R^4$ | Wasserstoff, Fluor, Chlor oder Methyl, bevorzugt in *ortho*-Position; |
| $R^{3'}$ und $R^{4'}$ | Wasserstoff; |
| $R^5$ und $R^6$ | unabhängig voneinander Wasserstoff oder Methyl; |
| $R^7$ und $R^8$ | unabhängig voneinander Methyl, Ethyl oder Methoxy bedeuten können. |

Ganz besonders bevorzugt sind Verbindungen der allgemeinen Formel **1**, worin

| | |
|---|---|
| $R^1$ und $R^2$ | unabhängig voneinander Wasserstoff, Methyl, Ethyl, Propyl, Butyl, Benzyl, Furylmethyl, Cyclopentyl, Cyclohexyl, Cyclopropylmethyl, Cyclobutylmethyl, Cyclopentylmethyl, Cyclohexylmethyl, $C_2$-$C_4$-Alkenyl, vorzugsweise Allyl, $C_2$-$C_4$-Alkinyl, vorzugsweise Propargyl, $C_1$-$C_4$-Alkoxy-$(CH_2)_l$- , $C_3$-$C_6$-Cycloalkoxy-$(CH_2)_m$-,<br>und<br>l eine ganze Zahl 1, 2 oder 3<br>und<br>m eine ganze Zahl 1, 2 oder 3,<br>oder |
| $R^1$ und $R^2$ | zusammen mit dem Stickstoffatom einen 5- oder 6-gliedrigen heterocyclischen Ring bilden, der gegebenenfalls mit 1 öder 2 Methylgruppen oder einer Dimethylengruppe substituiert sein kann; |
| n | 1; |
| $R^3$ | *ortho*-Fluor, *ortho*-Chlor oder *ortho*-Methyl; |
| $R^4$ | Wasserstoff, *ortho*-Fluor, *ortho*-Chlor oder *ortho*-Methyl; |
| $R^{3'}$ und $R^{4'}$ | Wasserstoff; |
| $R^5$ und $R^6$ | Wasserstoff; |
| $R^7$ und $R^8$ | gleich oder verschieden, Methyl oder Ethyl, bevorzugt Methyl bedeuten können. |

Als erfindungsgemäß von besonderem Interesse seien u.a. die folgenden Verbindungen genannt:

- N-Pentamethylen-3-(2,6-difluorphenyl)methoxy-2-(2,6-dimethylphenyl)-propylamin;
- N-Pentamethylen-3-(2-fluorphenyl)methoxy-2-(2,6-dimethylphenyl)-propylamin;
- N-Pentamethylen-3-(2,6-dichlorphenyl)methoxy-2-(2,6-dimethylphenyl)-propylamin;
- N-Pentamethylen-3-(2,6-dimethylphenyl)methoxy-2-(2,6-dimethylphenyl)-propylamin;
- N-Cyclopropylmethyl-3-(2,6-difluorphenyl)methoxy-2-(2,6-dimethylphenyl)-propylamin;
- N-Allyl-3-(2,6-difluorphenyl)methoxy-2-(2,6-dimethylphenyl)-propylamin;
- N-(3,3-Dimethylallyl)-3-(2,6-difluorphenyl)methoxy-2-(2,6-dimethylphenyl)-propylamin;
- N-(2-Methylallyl)-3-(2,6-difluorphenyl)methoxy-2-(2,6-dimethylphenyl)-propylamin;
- N-(1-Methylallyl)-3-(2,6-difluorphenyl)methoxy-2-(2,6-dimethylphenyl)-propylamin;

Gegenstand der Erfindung sind die jeweiligen Verbindungen gegebenenfalls in Form der einzelnen optischen

Isomeren, Mischungen der einzelnen Enantiomeren oder Racemate sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie z. B. Oxal-, Fumar- oder Diglycolsäure oder Methansulfonsäure.

Soweit nicht im einzelnen abweichende Angaben gemacht werden, werden die allgemeinen Definitionen im folgenden Sinn gebraucht:

$C_1$-$C_4$-Alkyl bzw. $C_1$-$C_8$-Alkyl steht im allgemeinen für einen verzweigten oder unverzweigten Kohlenwasserstoffrest mit 1 bis 4 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:

Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl, Ethyl, n-Propyl, iso-Propyl, n-Butyl, 1-Methylpropyl, 2-Methylpropyl oder 1,1-Dimethylethyl. Dabei sind von den Definitionen Propyl, Butyl, Pentyl etc. die jeweiligen isomeren Reste stets mit umfaßt.

Entsprechend bedeutet Alkylen eine verzweigte oder unverzweigte zweibindige Kohlenwasserstoffbrücke mit 1 bis 8 Kohlenstoffatomen, die gegebenenfalls mit einem oder mehreren Halogenatom(en) - vorzugsweise Fluor - substituiert sein kann, die untereinander gleich oder verschieden sein können.

Cycloalkyl steht im allgemeinen für einen gesättigten oder ungesättigten cyclischen Kohlenwasserstoffrest mit 3 bis 9 Kohlenstoffatomen, der gegebenenfalls mit einem Halogenatom oder mehreren Halogenatomen - vorzugsweise Fluor - substituiert sein kann, die untereinander gleich oder verschieden sein können. Bevorzugt sind cyclische Kohlenwasserstoffe mit 3 bis 6 Kohlenstoffatomen. Als Beispiele seien Cyclopropyl, Cyclobutyl, Cyclopentyl, Cyclopentenyl, Cyclohexyl, Cyclohexenyl, Cycloheptyl, Cycloheptenyl, Cycloheptadienyl, Cyclooctyl, Cyclooctenyl, Cyclooctadienyl und Cyctononinyl genannt.

Alkenyl verkörpert im allgemeinen einen verzweigten oder unverzweigten Kohlenwasserstoffrest mit 2 bis 8 Kohlenstoffatomen, der eine oder mehrere Doppelbindungen aufweisen kann und der gegebenenfalls durch ein oder mehrere Halogenatome - vorzugsweise Fluor - substituiert sein kann, wobei die Halogene untereinander gleich oder verschieden sein können. Folgende Alkenylreste seien beispielhaft genannt:

Vinyl, 2-Propenyl (Allyl), 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-Pentenyl, 4-Methyl-2-pentenyl, 1-Methyl-3-pentenyl, 2-Methyl-3-pentenyl, 3-Methyl-3-Pentenyl, 4-Methyl-3-pentenyl, 1-Methyl-4-pentenyl, 3-Methyl-4-pentenyl, 4-Methyl-4-pentenyl, 1,1-Dimethyl-2-butenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl.

Unter den Niederalkenylresten, die drei oder vier Kohlenstoffatome und eine Doppelbindung aufweisen, ist der Allylrest bevorzugt.

Alkinyl verkörpert im allgemeinen einen verzweigten oder unverzweigten Kohlenwasserstoffrest mit 2 bis 8 Kohlenstoffatomen, der eine oder mehrere Dreifachbindungen aufweisen kann und der gegebenenfalls durch ein oder mehrere Halogenatome - vorzugsweise Fluor - substituiert sein kann, wobei die Halogene untereinander gleich oder verschieden sein können. Folgende Alkinylreste seien beispielhaft genannt:

2-Propinyl (Propargyl), 2-Butinyl, 3-Butinyl, 1-Methyl-2-propinyl, 2-Methyl-2-propinyl, 2-Pentinyl, 3-Pentinyl, 4-Pentinyl, 1-Methyl-2-butinyl, 2-Methyl-2-butinyl, 3-Methyl-2-butinyl, 1-Methyl-3-butinyl, 2-Methyl-3-butinyl, 3-Methyl-3-butinyl, 1,1-Dimethyl-2-propinyl, 1,2-Dimethyl-2-propinyl, 1-Ethyl-2-propinyl, 2-Hexinyl, 3-Hexinyl, 4-Hexinyl, 5-Hexinyl, 1-Methyl-2-pentinyl, 2-Methyl-2-pentinyl, 3-Methyl-2-pentinyl, 4-Methyl-2-pentinyl, 1-Methyl-3-pentinyl, 2-Methyl-3-pentinyl, 3-Methyl-3-pentinyl, 4-Methyl-3-pentinyl, 1-Methyl-4-pentinyl, 3-Methyl-4-pentinyl, 4-Methyl-4-pentinyl, 1,1-Dimethyl-2-butinyl, 1,1-Dimethyl-2-butinyl, 1,1-Dimethyl-3-butinyl, 1,2-Dimethyl-2-butinyl, 1,2-Dimethyl-3-butinyl, 1,3-Dimethyl-2-butinyl, 1,3-Dimethyl-3-butinyl, 2,2-Dimethyl-3-butinyl, 2,3-Dimethyl-2-butinyl, 2,3-Dimethyl-3-butinyl, 1-Ethyl-2-butinyl, 1-Ethyl-3-butinyl, 2-Ethyl-1-butinyl, 2-Ethyl-2-butinyl, 2-Ethyl-3-butinyl, 1,1,2-Trimethyl-2-propinyl, 1-Ethyl-1-methyl-2-propinyl und 1-Ethyl-2-methyl-2-propinyl.

Unter den Niederalkinylresten, die drei oder vier Kohlenstoffatome und eine Dreifachbindung aufweisen, ist der Propargylrest bevorzugt.

Alkoxy steht im allgemeinen für einen über ein Sauerstoffatom gebundenen geradkettigen oder verzweigten

Kohlenwasserstoffrest - bevorzugt ist ein Niederalkoxyrest mit 1 bis 4 Kohlenstoffatom(en). - Besonders bevorzugt ist die Methoxygruppe.

Unter einem ankondensierten aromatischen Ring ist, soweit nicht anders definiert, erfindungsgemäß bevorzugt ein ankondensierter Benzolring zu verstehen.

## Biologische Eigenschaften

Die beanspruchten Verbindungen sind Blocker des spannungsabhängigen Natriumkanals. Es handelt sich dabei um Verbindungen, die Batrachotoxin (BTX) mit hoher Affinität ($K_i$ < 1000 nM) kompetitiv oder nicht-kompetitiv von der Bindungsstelle am Natriumkanal verdrängen. Solche Substanzen zeigen eine "usedependency" bei der Blockade der Natriumkanäle, d.h. für die Bindung der Substanzen an den Natriumkanal müssen die Natriumkanäle zunächst aktiviert werden. Die maximale Blockade der Natriumkanäle wird erst nach wiederholter Stimulation der Natriumkanäle erreicht. Demzufolge binden die Substanzen bevorzugt an Natriumkanäle, die vermehrt aktiviert werden. Dadurch sind die Substanzen in der Lage, bevorzugt in den Körperregionen wirksam zu werden, die pathologisch überstimuliert sind. Die erfindungsgemäßen Verbindungen der allgemeinen Formel **1** können somit bei Erkrankungen, deren Ursache auf eine durch Übererregung bedingte Funktionsstörung beruhen, eingesetzt werden. Darunter fallen Erkrankungen wie Arrhythmien, Spasmen, kardiale und Gehimischämien, Schmerzen sowie neurodegenerative Erkrankungen verschiedener Genese. Als Beispiele seien genannt: Epilepsie, Hypoglykämie, Hypoxie, Anoxie, Gehimtrauma, Gehimoedem, Gehim-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischer Schmerz, neuropathischer Schmerz sowie Lokalanaesthesie.

Als Testsystem für den Nachweis der Natriumkanal blockierenden Wirkung dient die BTX-Bindung am Natriumkanal [S.W. Postma & W.A. Catterall, Mol. Pharmacol. 25, 219-227 (1984)] sowie patch-clamp Experimente, in denen gezeigt werden kann, daß die erfindungsgemäßen Verbindungen den elektrisch stimulierten Natriumkanal in einer "use-dependent" Art und Weise blockieren [W.A. Catterall, Trends Pharmacol. Sci., 8, 57-65 (1987)]. Durch die Auswahl des Zellsystems (z.B. neuronale, kardiale, DRG Zellen) kann die Wirkung der Substanzen auf verschiedene Natriumkanalsubtypen untersucht werden.

Die Natriumkanal blockierende Eigenschaft der erfindungsgemäßen Verbindungen kann durch die Blockade des Veratridin induzierten Glutamat-releases nachgewiesen werden [S. Villauneva, P. Frenz, Y. Dragnic, F. Orrego, Brain Res. 461, 377-380 (1988)]. Veratridin ist ein Toxin, das den Natriumkanal permanent öffnet. Dadurch kommt es zu einem erhöhten Einstrom von Natriumionen in die Zelle. Über die oben beschriebene Kaskade führt dieser Natriumeinstrom in neuronalem Gewebe zu einer gesteigerten Freisetzung von Glutamat. Durch die erfindungsgemäßen Verbindungen läßt sich diese Glutamatfreisetzung antagonisieren.

Antikonvulsive Eigenschaften der erfindungsgemäßen Substanzen wurden durch eine protektive Wirkung gegen Krämpfe, die durch einen maximalen Elektroschock in Mäusen ausgelöst wurden, belegt [M. A. Rogawski & R.J. Porter, Pharmacol. Rev. 42, 223-286 (1990)].

Neuroprotektive Eigenschaften wurden durch protektive Wirkung in einem Ratten-MCAO-Modell [U. Pschom & A. J. Carter, J. Stroke, Cerebrovascular Diseases, 6, 93-99 (1996)] sowie einem Malonat induziertem Läsionsmodel [M.F. Beal, Annals of Neurology, 38, 357-366 (1995) und J.B. Schulz, R.T. Matthews, D.R. Henshaw und M.F. Beal, Neuroscience, 71, 1043-1048 (1996)] belegt.

Analgetische Wirkung lassen sich in Modellen der diabetischen Neuropathie sowie in einem Ligatur-Modell belegen [C. Courteix, M. Bardin, C. Chantelauze, J. Lavarenne, A. Eschalier, Pain 57, 153-160 (1994); C. Courteix, A. Eschalier, J. Lavarenne, Pain 53, 81-88 (1993); G. J. Bennett & Y.-K. Xie, Pain 33, 87-107 (1988)].

Ferner wurde beschrieben, daß Natriumkanalblocker zur Therapie der Zyklophrenie (manisch depressive Erkrankung) eingesetzt werden können [J. R. Calabrese, C. Bowden, M.J. Woyshville; in: Psychopharmacology: The Fourth Generation of Progress (Eds.: D. E. Bloom and D. J. Kupfer) 1099-1111. New York: Raven Press Ltd.].

## Herstellungsverfahren

Die beanspruchten Verbindungen 1 lassen sich nach an sich aus dem Stand der Technik bekannten Verfahren herstellen. Ein möglicher Syntheseweg ist in Schema 1 dargestellt. Ausgangsverbindung sind die substituierten Benzylcyanide vom Typ der allgemeinen Formel **2**. Deratige Benzylcyanid-Derivate - wie z.B. 2,6-Dimethylbenzylcyanide der allgemeinen Formel **2** sowie ihre Herstellung sind in der Literatur beschrieben [z.B. Bennett et al., J. Med. Chem. **24,** 382-389 (1981); Benington et al. J. Org. Chem. **23**, 2034-2035 (1958); Carlin et al. J. Org. Chem. 30, 563-566 (1965)], oder lassen sich analog nach diesen Verfahren herstellen.

Schema 1 (die N-Schutzgruppe SG ist hier beispielhaft als BOC-Schutzgruppe dargestellt):

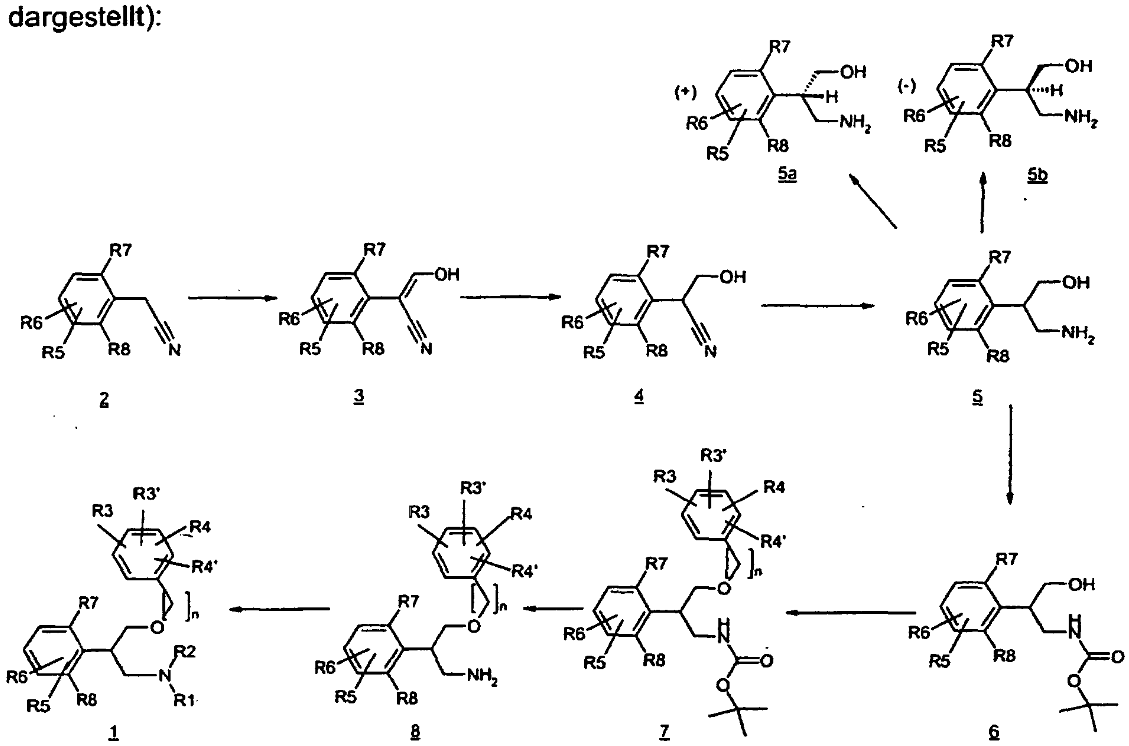

Die 2,6-substituierten Benzylcyanid-Derivate der allgemeinen Formel **2** werden nach Deprotonierung mit Ameisensäureester - vorzugsweise mit Ameisensäureethylester - umgesetzt. Die Deprotonierung kann dabei mit allen geeigneten aus dem Stand der Technik bekannten Basen erfolgen. Vorzugsweise werden hierbei Alkaliorganyle als metallorganische Basen - insbesondere Alkalialkoholate - eingesetzt; besonders bevorzugt wird Kalium-*tert*.-butylat zur Deprotonierung verwandt. Als Reaktionsmedien können alle aprotischen Lösungsmittel eingesetzt werden, die sich unter den gegebenen Reaktionsbedingungen inert verhalten und die sich von ihren physikalischen Parametern her für die Deprotonierungsreaktion eignen. Als Lösungsmittel werden insbesondere aliphatische oder aromatische Kohlenwasserstoffe eingesetzt, worunter - gegebenenfalls alkylsubstituierte - Aromaten bevorzugt werden; besonders bevorzugt wird Toluol als Reaktionsmedium eingesetzt. Die Reaktionstemperatur liegt in einem Bereich, der einerseits nicht zu niedrig ist, um die gewünschte Deprotonierung zu erschweren bzw. zu unterdrücken, die jedoch nicht in einem Bereich liegt, welche das Auftreten von Neben- bzw. Folgereaktionen ermöglicht. In Abhängigkeit von dem jeweils eingesetzten Lösungsmittel wird eine Temperatur in einem Intervall von -20 bis +30 °C - bevorzugt in einem Intervall von -10 bis 20 °C und besonders bevorzugt in einem Intervall von -10 bis -5 °C - gewählt, wobei zweckmäßigerweise bei Raumtemperatur (ca. 0 bis 30 °C) nachreagieren gelassen wird. Nach abschließender Hydrolyse wird das Reaktionsprodukt vom Typ **3** beispielsweise auf dem Wege der Extraktion isoliert und gegebenenfalls mit an sich aus dem Stand der Technik bekannten Standardmethoden - vorzugsweise mittels Umkristallisation - gereinigt.

Anschließend wird die Formylgruppe zum Alkohol der allgemeinen Formel **4** und nachfolgend das Nitril **4** zum Amin **5** reduziert. Die Reduktion der Formlygruppe zum entsprechenden Alkohol ist aus dem Stand der Technik wohlbekannt [J. March, Advanced Organic Chemsitry, Wiley Interscience, New York (1989), 4. Auflage, Seite 910 ff.; C Larock, Comprehensive Organic Transformations, VCH Publishers Inc., New York (1989), S. 527 ff und zit. Lit.]. Bevorzugt wird die Reduktion mit komplexen Hydriden - wie z.B. mit komplexen Alkalibor- oder Alkalialuminiumhydriden oder gegebenenfalls mit deren geeigneten Derivaten, worunter der Einsatz von Natriumcyanoborhydrid besonders bevorzugt wird. Dabei ist es in der Regel vorteilhaft, derartige Reduktionen in der Gegenwart eines Überschusses an Reduktionsmittel, welches vorzugsweise eines der vorgenannte Hydride verkörpert, ablaufen zu lassen, der in einem Bereich von 5 bis 100 % und vorzugsweise in einem Bereich von 50 bis 100 % und besonders bevorzugt in einem Bereich von 70 bis 90 % liegt. Als Reaktionsmedien eignen sich alle Lösungsmittel, die den Reaktionsverlauf nicht nachteilig beeinflussen. Derartige Lösemittel sind aus dem Stand der Technik hinreichend bekannt; vorzugsweise werden verzweigte oder unverzweigte Alkohole - insbesondere niedere $C_1$-$C_4$-Alkanole eingesetzt -, worunter Methanol besonders bevorzugt wird. Die Reduktion kann in einem weiten Temperaturbereich durchgeführt werden, wobei sich die jeweilige Temperatur - neben den physikalischen Parametern des Reaktionsmediums - insbesondere nach der

Aktivität des jeweils eingesetzten komplexen Hydrids richtet. Die Isolierung des Reduktionsproduktes **4** erfolgt nach Zerstörung des überschüssigen Reduktionsmittel auf an sich aus dem Stand der Technik bekannte Weise - insbesondere auf dem Wege der Extraktion.

Die Reduktion des so erhaltenen Nitrits **4** zu dem entsprechenden Amin ist an sich ebenfalls aus dem Stand der Technik bekannt [J. March, Advanced Organic Chemsitry, Wiley Interscience, New York (1985), 3. Auflage, Seite 815 ff.; C. Larock, Comprehensive Organic Transformations, VCH Publishers Inc., New York (1989), S. 437 ff. und zit. Lit.; P.N. Rylander: Hydrogenation Methods, Academic Press, New York (1985), Kapitel 7]. Erfindungsgemäß wird die katalytische Reduktion mit Wasserstoff in Gegenwart von Raney-Nickel in Gegenwart eines Amins - vorzugsweise in Gegenwart von Ammoniak - bevorzugt. Als Reaktionsmedium können bei der Reduktion alle Lösungsmittel dienen, die den Reaktionsvertauf bzw. insbesondere die Aktivität des Katalysators nicht nachteilig beeinflussen. Derartige Lösemittel sind aus dem Stand der Technik in ausreichender Zahl bekannt; vorzugsweise werden verzweigte oder unverzweigte Alkohole - insbesondere niedere $C_1$-$C_4$-Alkanole eingesetzt -, worunter Methanol besonders bevorzugt wird. Die übrigen Parameter der Reduktionsreaktion können in weiten Bereichen variiert werden und sind neben dem Edukt insbesondere von der Aktivität des vorzugsweise eingesetzten Raney-Nickels abhängig. Vorzugsweise wird die Reduktion unter einem Wasserstoffdruck in einem Bereich von 10 bis 200 bar durchgeführt, wobei ein Wasserstoffdruck von 70 bar besonders bevorzugt wird. Die Reaktionstemperatur kann in einem Intervall von 20 bis 150 °C gewählt werden - besonders bevorzugt wird die Reduktion bei 70 °C durchgeführt. Nach vollständiger Reduktion und gegebenenfalls nach dem Abkühlen des Reaktionsgemisches wird der Katalysator abfiltriert und das Filtrat mit an sich aus dem Stand der Technik bekannten Methoden - wo möglich auf dem Wege der Destillation (im Hochvakuum) gereinigt.

Auf der Stufe des Aminoalkohols **5** kann das Racemat gegebenenfalls in die Enantiomeren gespalten werden. Dabei kann die anschließende Aufspaltung des so erhaltenen Gemisches der enantiomeren Aminoalkohole vom Typ **5** auf den an sich aus dem Stand der Technik bekannten Wegen zur Enantiomerentrennung - beispielsweise durch Umsetzung mit Äpfelsäure, Weinsäure, Mandelsäure oder Campfersulfonsäure, worunter Weinsäure besonders bevorzugt ist, erfolgen.

So liefert beispielsweise die Umsetzung mit S-(-)-Weinsäure im Falle des 3-Hydroxy-2-(2,6-dimethylphenyl)-propylamins den entsprechenden enantiomerenreinen Aminoalkohol vom Typ **5a** in Form seines Hydrogentartrates; analog liefert die entsprechende Umsetzung mit R-(+)-Weinsäure den enantiomerenreinen Aminoalkohol vom Typ **5b**.

Zur Isomerentrennung - beispielsweise über die entsprechenden Tartrate - wird der Aminoalkohol **5** - beispielsweise in Form der freien Base - in einem verzweigten oder unverzweigten $C_1$-$C_4$-Alkanol, besonders bevorzugt in Methanol - gelöst und mit dem geeigneten Stereoisomeren einer der genannten Säuren - beispielsweise D-(-)-Weinsäure - versetzt. Erforderlichenfalls wird eine ausreichende Menge eines Nichtlösemittels bezüglich des gewünschten Salzes - vorzugsweise des entsprechenden Hydrogentartrates zugefügt, worauf das enantiomerenreine Isomere des Aminoalkohols **5** als Hydrogentartrat kristallisiert, das - notwendigenfalls - auf dem Wege der Umkristallisation weiter aufgereinigt werden kann.

Der racemische oder enantiomerenreine Aminoalkohol **5** bzw. **5a** oder **5b** wird auf der nächsten Stufe mit einer Verbindung vom Typ X-S, worin X eine gegen einen Aminstickstoff substituierbare Austrittsgruppe und S eine für den Schutz von primären Aminen geeignete Schutzgruppe verkörpert, umgesetzt. Derartige Reagenzien sowie Methoden zu ihrer Anbindung von Schutzgruppen an Amine sind aus dem Stand der Technik in großer Zahl bekannt [T.W. Greene und P.G.M. Wuts: Protective Groups in Organic Synthesis, John Wiley & Sons, Inc., New York (1991), S. 309 ff.]. Erfindungsgemäß wird im vorliegenden Fall der Aminstickstoff vorzugsweise unter Verwendung von Pyrokohlensäuredi-*tert*.-butylester selektiv N-geschützt. Dazu wird der racemische oder enantiomerenreine Aminoalkohol vom Typ **5** bzw. **5a** oder **5b** in einem unter den gegebenen Reaktionsbedingungen inerten Lösungsmittel gelöst. Als Lösungsmittel eignen sich vorzugsweise Niederalkylester niederer Carbonsäuren, worunter Essigsäureethylester besonders bevorzugt wird. Die Umsetzung mit dem Pyrokohlensäuredi-*tert*.-butylester erfolgt dabei vorzugsweise in einem Temperaturintervall von -20 bis 75 °C und besonders bevorzugt in einem Bereich von -10 bis +25 °C. Nach erfolgter Umsetzung wird das Lösungsmittel abgezogen, der Rückstand mit der wässerigen Lösung einer sauer reagierenden Verbindung - vorzugsweise mit 90 proz. Essigsäure - versetzt. Nach circa einer Stunde wird das Reaktionsgemisch eingeengt und der Rückstand mit einem geeigneten Lösungsmittel, vorzugsweise mit einem Niederalkylester einer niederen Carbonsäure und besonders bevorzugt mit Essigsäureethylester aufgenommen und nach dem Waschen mit einer basisch reagierenden Waschlösung - vorzugsweise mit wässeriger Ammoniaklösung - vom Lösungsmittel befreit.

Anschließend wird im N-geschützten Aminoalkohol vom Typ **6** im Alkalischen die Alkoholfunktion mit entsprechend substituierten Phenylalkylhalogeniden umgesetzt, woraus die gewünschten Etherstrukturen der allgemeinen Formel **7** resultieren. Die Umsetzungen von Alkoholen des Typs **6** mit Phenylalkylhalogeniden - insbesondere mit Benzylhalogeniden - ist aus dem Stand der Technik wohlbekannt [C. Larock, Comprehensive Organic Transformations, VCH Publishers Inc., New York, Weinheim (1989), S. 446 ff. und zit. Lit.].

Zur Herstellung der Aminoether vom Typ **7** wird beispielsweise der geschützte Aminoalkohol der allgemeinen Formel **6** in einem geeigneten, unter den gewählten Reaktionsbedingungen inerten Lösungsmittel gelöst. Als Reaktionsmedien

kommen bei der erfindungsgemäßen Durchführung der Reaktion halogenierte niedere Kohlenwasserstoffe, worunter halogenierte $C_1$- oder $C_2$- Alkane bevorzugt werden, worunter Methylenchlorid (Dichlormethan) als Reaktionsmedium besonders bevorzugt wird, in Frage. Bei der Wahl der vorgenannten Lösungsmittel hat sich der Einsatz eines Phasentransfer-Katalysators als besonders vorteilhaft herausgestellt.

Derartige Phasentransfer-Katalysatoren sind aus dem Stand der Technik in hinreichender Zahl bekannt [Römpp, Lexikon Chemie, Georg Thieme Verlag, Stuttgart (1998)]. Bei der Herstellung der erfindungsgemäßen Verbindungen haben - sich sog. Tetraalkylammonium-Verbindungen als besonders geeignet erwiesen. Unter derartigen Phasentansfer-Katalysatoren werden erfindungsgemäß quartäre Ammonium-Verbindungen des Typs $[R_4N]+X-$ verstanden, in denen die Substituenten R, die gleich oder verschieden sein können, vorzugsweise Niederalkylreste verkörpern. Erfindungsgemäß besonders bevorzugt sind Tetrabutylammoniumsalze, worunter Tetra-n-butylammoniumhydrogensulfat besonders bevorzugt wird. Als wässerige Phase dient dabei vorzugsweise eine wässerige Lösung einer basisch reagierenden Verbindung eines Alkali- oder Erdalkalimetalls. Bevorzugt wird der Einsatz einer wässerigen Lösung eines Alkalihydroxids, worunter 50-proz. wässerige Natronlauge besonders bevorzugt wird. Die Umsetzung mit dem Phenylalkylderivat, vorzugsweise mit einem Phenylalkylhalogenid und besonders bevorzugt mit einem Phenylalkylbromid, kann in einem weiten Temperaturbereich erfolgen, der hinsichtlich der Untergrenze durch die Reaktionsfähigkeit der Reaktanden und hinsichtlich der Obergrenze durch den Siedepunkt des jeweils eingesetzten Lösungsmittels bestimmt wird. Bevorzugt wird die Substitutionsreaktion bei einer Temperatur in einem Intervall von +5 bis 60 °C und besonders bevorzugt bei 0 bis 30° C (das entspricht im Sinne der vorliegenden Erfindung Raumtemperatur) durchgeführt.

Nach erfolgter Durchführung wird die organische Phase separiert und die wässerige Phase erschöpfend mit einem geeigneten Lösungsmittel - vorzugsweise mit einem halogenierten niederen Kohlenwasserstoff, worunter halogenierte $C_1$- oder $C_2$-Alkane besonders bevorzugt werden und ganz besonders bevorzugt mit Methylenchlorid extrahiert. Die vereinigten organischen Phasen werden anschließend mit der wässerigen Lösung einer sauer reagierenden Verbindung, vorzugsweise mit der wässerigen Lösung einer Mineralsäure und besonders bevorzugt mit 2 N Salzsäure gewaschen, getrocknet und eingeengt. Der Rückstand kann dann weiter mit an sich aus dem Stand der Technik bekannten Verfahren - insbesondere auf dem Wege der Kristallisation - weiter aufgereinigt werden.

Nach Abspaltung der Schutzgruppe mit HCl-Gas können dann wahlweise über Alkylierung, reduktive Aminierung oder Acylierung sowie anschließender Reduktion die Reste $R_1$ und $R_2$ eingeführt werden.

Zur Abspaltung der Schutzgruppe vom Amin-Stickstoff ist anzumerken, daß diese ebenfalls aus dem Stand der Technik bekannt ist [T.W. Greene und P.G.M. Wuts: Protective Groups in Organic Synthesis, John Wiley & Sons, Inc., New York (1991), S. 309 ff.]. Die darauf folgende Einführung der Reste $R_1$ und $R_2$ an der AminoFunktion kann zum einen im Rahmen einer Acylierung mit nachfolgender Reduktion erfolgen. Die dazu erforderlichen Carbonsäurederivate sind entweder aus dem Stand der Technik bekannt oder sind auf dem Wege gängiger Syntheseverfahren leicht zugänglich [Houben-Weyl: Methoden der organischen Chemie, Band VIII und Band E5, Georg Thieme Verlag, Stuttgart 1952 bzw. 1985].

Für die Acylierung selbst stehen wiederum eine Vielzahl von Verfahren zur Auswahl [C. Ferri: Reaktionen der organischen Synthese, Georg Thieme Verlag, Stuttgart (1978), S.222 ff. und zit. Lit; J. March, Advanced Organic Chemistry, 3rd Edition., John Wiley and Sons, New York 1985, S. 370 ff. und zit. Lit.; R.C. Larock, Comprehensive Organic Transformations - A Guide to Functional Group Preparations, VCH Verlagsgesellschaft, Weinheim (1989), s. 963 ff. und zit. Lit.], wobei Umsetzungen mit Carbonsäurehalogeniden in einem unter den herrschenden Reaktionsbedingungen im wesentlichen inerten Lösungsmittel - gegebenenfalls in Gegenwart von säurebindenden Agenzien - wie z.B.: tertiären Aminen oder Alkalioder Erdalkalisalzen - bevorzugt werden [A.L.J. Beckwith in J. Zabicki: The Chemistry of Amides, Interscience, New York (1970), S. 73 ff.]. Als inerte Lösungsmittel werden im allgemeinen organische Solvenzien eingesetzt, die sich unter den angewandten Reaktionsbedingungen nicht verändern, wie z.B.:

Kohlenwasserstoffe - beispielsweise Benzol, Toluol, Xylol oder Erdölfraktionen - oder Ether - wie z.B.: Diethylether, Glykoldimethylether (Glyme), Diglykoldimethylether (Diglyme) - oder cyclische Ether - wie z.B.: Tetrahydrofuran (THF), oder Dioxan - oder halogenierte Kohlenwasserstoffe - wie beispielsweise Dichlormethan (Methylenchlorid).

Zweckmäßigerweise wird der Aminoether der allgemeinen Formel **8** bevorzugt in halogenierten Kohlenwasserstoffen - besonders bevorzugt in THF - in Gegenwart von säurebindenden Alkali- oder Erdalkalicarbonaten - besonders bevorzugt in Gegenwart von Kaliumcarbonat mit dem gewünschten Säurehalogenid - vorzugsweise mit dem entsprechenden Säurechlorid - umgesetzt.

Es ist jedoch auch möglich, die Umsetzung - in Anlehnung an die sog. Schotten-Baumann-Variante - in Wasser oder in einem wässerigen Alkohol in Gegenwart von Alkalihydroxiden oder Alkalicarbonaten durchzuführen [Organikum, Organischchemisches Grundpraktikum, 19. Aufl., Johann Ambrosius Barth, Leipzig, Edition Deutscher Verlag der Wissenschaften (1993), S. 424]. - In Abhängigkeit von den eingesetzten Edukten kann es sich des weiteren als vorteilhaft erweisen, die Acylierung nach der Variante von Einhorn durchzuführen [Organikum, Organischchemisches Grundpraktikum, 19. Aufl., Johann Ambrosius Barth, Leipzig, Edition Deutscher Verlag der Wissenschaften (1993), S. 424], wobei Pyridin sowohl als säurebindendes Agens wie auch als Reaktionsmedium eingesetzt wird.

Des weiteren besteht die Möglichkeit, die Acylierungsreaktion mit der jeweiligen freien Carbonsäure durchzuführen [A.L.J. Beckwith in J. Zabicki,: The Chemistry of Amides, interscience, New York (1970), S. 105 ff.; J.A. Mitchell und E.E. Reid, J. Am. Chem. Soc. 53, (1931) 1879]. - Des weiteren kann es sich als zweckmäßig erweisen, ein gemischtes Anhydrid - z.B. mit einem Kohlensäureester - einzusetzen [C. Ferri: Reaktionen der organischen Synthese, Georg Thieme Verlag, Stuttgart (1978), S.222 ff. und zit Lit.; A.L.J. Beckwith in J. Zabicki,: The Chemistry of Amides, Interscience, New York (1970), S. 86 ff.; J. March, Advanced Organic Chemistry, 3rd Edition., John Wiley and Sons, New York 1985, S. 371 und zit. Lit.; R.C. Larock, Comprehensive Organic Transformations - A Guide to Functional Group Preparations, VCH Verlagsgesellschaft, Weinheim (1989), S. 981 ff. und zit. Lit.]. Bei der bevorzugten Acylierung mit Carbonsäurehalogeniden - insbesondere mit Carbonsäurechloriden - kann die Reaktionstemperatur in weiten Grenzen variiert werden, die nach unten durch zu geringe Reaktionsgeschwindigkeiten und nach oben durch das überhandnehmen von unerwünschten Nebenreaktionen gesteckt werden. Bei der praktischen Durchführung haben sich Reaktionstemperaturen in einem Intervall von -50 bis 150 °C und bevorzugt in einem Intervall von 0 bis 75 °C bewährt, wobei sich die gewählte Reaktionstemperatur natürlich auch nach dem ausgewählten Lösungsmittel richtet. Als Lösungsmittel kommen dabei in erster Linie inerte Lösungsmittel in Betracht, die die Acylierungsreaktion nicht nachteilig beeinflussen. Hierzu zählen in erster Linie Ether - wie z.B.: Diethylether, Glykoldimethylether (Glyme), Diglykoldimethylether (Diglyme) - oder cyclische Ether - wie z.B.: Tetrahydrofuran (THF), oder Dioxan, worunterTHF besonders bevorzugt wird. Dabei arbeitet man zweckmäßigerweise mit einem geringen Überschuß des Acylierungsmittels in Gegenwart eines - in etwas größerem Überschuß vorhandenem - säurebindenden Mittels, um eine möglichst vollständige Umsetzung der Edukte zu gewährleisten.

Um zu dem gewünschten Aminoether der allgemeinen Formel **1** zu gelangen ist es letztendlich erforderlich, im anschließenden Reaktionsschritt das so erhaltene Säureamid zu reduzieren. - Derartige Reduktionen von Säureamiden sind aus dem Stand der Technik ebenfalls wohlbekannt und können sowohl auf dem Wege der elektrolytischen Reduktion, durch Reduktion mit Alkalimetallen sowie durch katalytische Reduktion erfolgen [R. Schröter in Houben-Weyl: Methoden der organischen Chemie, Band XI/1 und Band E5, Georg Thieme Verlag, Stuttgart 1957, S. 574] oder mit Diboran bzw. Borwasserstoff-Derivaten [J. Furhop und G. Penztin, Organic Synthesis - Concepts - Methods - Starting Materials -, VCH-Verlagsgesellschaft, Weinheim 1986, S. 90] erfolgen.

Bevorzugt ist allerdings die Reduktion mit komplexen Hydriden, wie Alkalibor- oder Alkalialuminiumhydriden bzw. mit deren geeigneten Derivaten - gegebenenfalls in Gegenwart eines Katalysators - [N.G. Gaylord: Reduction with Complex Metal Hydrides, Wiley, New York (1965); A. Häjos: Complex Hydrides, Elsevier, New York (1979); V. Bazant, M. Capka, M. Cerny, V. Chvalovsky, K. Kochloefl, M. Kraus um M. Màlek, Tetrahedron Lett. 9 (1986) 3303], wobei die Verwendung von Lithiumaluminiumhydrid besonders bevorzugt wird.

Als Reaktionsmedien eignen sich hierbei alle inerten organischen Lösungsmittel, die sich unter den gegebenen Reaktionsbedingungen nicht verändern. Hierzu gehören bevorzugt Ether - wie beispielsweise Diethylether, Diisopropylether, Glykoldimethylether (Glyme), Diglykoldimethylether (Diglyme) - oder cyclische Ether - wie z.B.: Tetrahydrofuran (THF), oder Dioxan, worunter THF besonders bevorzugt wird, wobei sich die Wahl des Reaktionsmediums unter anderem nach der Art des eingesetzten Reduktionsmittels richtet.

Bei der Durchführung der Reaktion ist es in der Regel vorteilhaft, derartige Reduktionen in Gegenwart eines Überschusses des Reduktionsmittels, welches vorzugsweise eines der vorgenannten komplexen Hydride verkörpert - insbesondere Lithiumtetrahydridoalanat - ablaufen zu lassen, der in einem Bereich von 5 bis 100 % - vorzugsweise in einem Bereich zwischen 10 bis 50 % liegt. Dabei werden die Reaktionspartner üblicherweise unter Eiskühlung oder bei Raumtemperatur zusammengegeben und anschließend - in Abhängigkeit von der Reaktivität der Edukte - auf eine Reaktionstemperatur in einem Intervall von 50 bis 150 °C erwärmt. Eine weitere Möglichkeit zur Herstellung der erfindungsgemäßen Aminoether der allgemeinen Formel **1** aus dem Precursor **8** besteht andererseits in der Umsetzung des Amins vom Typ **8** mit geeigneten Alkylierungsmitteln. Derartige Alkylierungsmittel vom Typ $R_1$-Z bzw. $R_2$-Z sollten zweckmäßigerweise eine geeignete Austrittsgruppe aufweisen, die durch den Amino-Stickstoff substituiert werden kann. Beispiele bevorzugte Austrittsgruppen vom Typ Z sind Halogene - wie vorzugsweise Chlor, Brom oder Iod - oder -O-$SO_2$-Aryl - wie z.B.: Tosylat - oder ein Alkylsulfonat vom Typ -O-$SO_2$-Alkyl - wie z.B.: Methansulfonat oder Halogenmethansulfonat oder Sulfat. - Entsprechende Alkylierungsagenzien sind entweder kommerziell erhältlich oder deren Herstellung ist aus dem Stand der Technik bekannt.

Als Lösungsmittel eignen sich alle inerten Lösungsmittel, die sich unter den gegebenen Reaktionsbedingungen im wesentlichen nicht verändern und die selbst nicht als reaktionsfähige Komponenten nachteilig das Reaktionsgeschehen beeinflussen können. Hierzu gehören bevorzugt Ether - wie beispielsweise Diethylether, Diisopropylether, Glykoldimethylether (Glyme), Diglykoldimethylether (Diglyme) - oder cyclische Ether - wie z.B.: Tetrahydrofuran (THF), oder Dioxan - oder Ketone - wie z.B.: Methylethylketon oder Aceton - oder Säureamide - wie Hexamethylphosphorsäuretriamid oder Dimethylformamid (DMF).

Außerdem ist es möglich, Gemische der genannten Lösungsmittel zu verwenden. Besonders bevorzugt werden THF oder Dimethylformamid (DMF) eingesetzt. Die Alkylierungsreaktion wird vorzugsweise in Gegenwart von säurebindenden Agenzien - wie z.B.: Alkali- oder Erdalkalicarbonaten oder -hydrogencarbonaten - durchgeführt.

Die Reaktionstemperatur läßt sich bei der Reaktionsführung in weiten Grenzen variieren, die für die praktische Durchführung - neben den entsprechenden physikalischen Größen des Lösungsmittels - nach unten durch eine zu geringe Reaktionsgeschwindigkeit und nach oben durch ein Überhandnehmen von Nebenreaktionen gesetzt werden. - Geeignete Reaktionstemperaturen liegen in einem Intervall von 0 bis 150 °C und - bevorzugt - zwischen 50 und 100 °C. Des weiteren besteht die Möglichkeit ,die gewünschten Substituenten an der Aminofunktion im Rahmen einer reduktiven Aminierung [Organikum, Organischchemisches Grundpraktikum, 19. Aufl., Johann Ambrosius Barth, Leipzig, Edition Deutscher Verlag der Wissenschaften (1993), S. 451; [C. Ferri: Reaktionen der organischen Synthese, Georg Thieme Verlag, Stuttgart (1978), S.85 ff.] - beispielsweise im Rahmen einer Leuckard-Wallach-Reaktion oder einer Aminierung nach Decker/Forster [H. Krauch und W. Kunz, Reaktionen der organischen Chemie, Hüthig Verlag, Heidelberg (1997) S. 104 ff.] einzuführen und so zu den Aminoethem der allgemeinen Formel 1 zu gelangen.
Bei der katalytischen reduktiven Aminierung findet im allgemeinen Raney-Nickel, welches gegebenenfalls mit weiteren Elementen - wie z.B. Chrom - dotiert ist Verwendung. Daneben kann die reduktive Aminierung auch in Gegenwart eines Platin-Katalysator durchgeführt werden, was normalerweise die Durchführung der Reaktion unter milderen Bedingungen ermöglicht. Im allgemeinen wird bei der katalytischen reduktiven Aminierung in einem Temperaturintervall von 20 bis 160 °C gearbeitet. Die einzustellende Temperatur hängt dabei im wesentlichen von der Aktivität des Katalysators, von der Reaktivität der Amin- und Carbonylkomponente ab. Als Lösungsmittel kommen in erster Linie Alkohole oder Wasser in Frage worunter niedere Alkohole - wie Methanol, Ethanol oder Isopropanol - bevorzugt werden; besonders bevorzugt wird Methanol als Lösungsmittel eingesetzt. Der Wasserstoffdruck kann ebenfalls in einem weiten Bereich variiert werden und liegt im allgemeinen in einem Intervall von 1 bis 100 atü (1.01 bis 101,33 bar) - vorzugsweise bei 5 bis 80 atü (5,07 bis 81.06 bar).

Alternativ können die beanspruchten Verbindungen 1, bei denen $R_1$ und $R_2$ = $-(CH_2)_m-$ (mit m vorzugsweise 5 oder 6) ist, über das in Schema 2 dargestellte Verfahren hergestellt werden.

Schema 2:

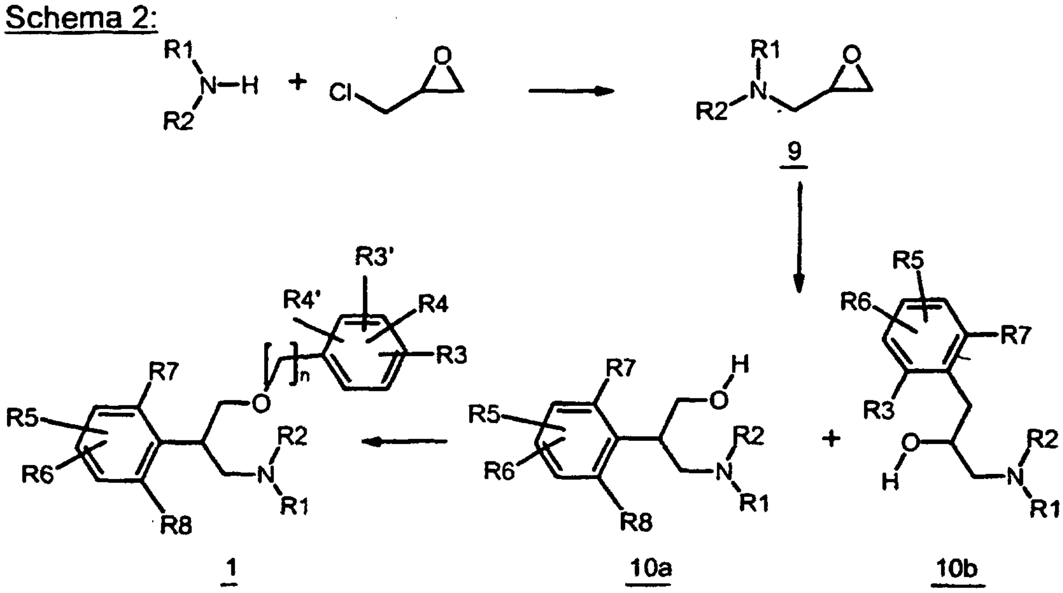

9

1 10a 10b

Die 3-stufige Reaktionsfolge kann durch Einsatz von racemischem Epichlorhydrin bzw. R- oder S-Epichlorhydrin wahlweise racemisch oder stereospezifisch - in Analogie zu aus dem Stand der Technik bekannten Methoden [J. Amer. Chem. Soc. 80 (1958) 1257] - durchgeführt werden. Die Aminooxiranzwischenstufen **9** werden zweckmäßigerweise destillativ oder durch Flash-Chromatographie aufgereinigt; sie können aber auch direkt als Rohprodukte weiterverarbeitet werden. Die Zwischenstufe **10a** wird durch Grignard-Reaktion generiert und zweckmäßigerweise vom nicht erwünschten Regioisomeren **10b** chromatographisch abgetrennt. Das Regioisomerenverhältnis kann zugunsten von **10a** verschoben werden, wenn das Aminooxiran **9** mit einem Equivalent $MgCl_2$-Etherat versetzt und der gebildete feinkristalline Niederschlag mit einem Equivalent Grignard-Reagens umgesetzt wird (inverse Zugabe). Als vorteilhaft, weil präparativ gegebenenfalls einfacher durchführbar und neben der erwünschten Regioisomerenverschiebung unter Umständen mit einer Ausbeutesteigerung verbunden, kann die Generierung eines Diaryl-Magnesium-Reagens (durch Fällen von $MgBr_2$ mit Dioxan) und dessen Umsetzung mit **9** zu **10a** erscheinen. Die Separierung der Regioisomeren **10** gelingt auch nach dem Verätherungsschritt bei den Endverbindungen der allgemeine Formel **1**. Die Variation der Kettenlänge (n) erfolgt z.B. durch Verwendung der Mitsunobu-Reaktion , durch Verwendung von Benzylhalogeniden bzw. Phenylalkylhalogeniden unter bevorzugtem Einsatz von Kalium-*tert.*-butylat als Hilfsbase und durch Reppe-Reaktion mittels gegebenenfalls substituierter Phenylacetylene und anschließender Hydrierung der entstehenden Z/E-Olefingemische.

Im folgenden sind einige Beispiele für pharmazeutische Zubereitungen mit dem Wirkstoff angegeben:

Tabletten:

| | |
|---|---|
| Wirkstoff gemäß allgemeiner Formel 1 | 20 mg |
| Magnesiumstearat - | 1 mg |
| Laktose | 190 mg |

Lösung zur Iniektion

| | |
|---|---|
| Wirkstoff gemäß allgemeiner Formel I | 0,3 mg |
| Natriumchlorid | 0,8 g |
| Benzalkoniumchlorid | 0,01 mg |
| Aqua ad injectionem ad 100 ml | |

Eine ähnliche Lösung zu der oben aufgeführten ist geeignet für die nasale Applikation in einem Spray, oder in Kombination mit einem Gerät, das ein Aerosol mit einer Partikelgröße vorzugsweise zwischen 2 und 6 μM, zur Anwendung über die Lunge.

Lösung zur Infusion

Eine 5 gewichtsprozentige Xylit- oder eine Kochsalzlösung, die beispielseise den Wirkstoff in einer Konzentration von 2 mg/ml enthält, wird mit einem Natriumacetat-Puffer auf einen pH-Wert von ca. 4 eingestellt.

Derartige Infusionslösungen können einen Gehalt an Wirkstoff gemäß der allgemeinen Formel 1 bezogen auf die Gesamtmasse der pharmazeutischen Zubereitung in einem Bereich von 0,001 bis 5 Gew.-%, vorzugsweise in einem Bereich von 0,001 bis 3 Gew.-% und besonders bevorzugt in einem Bereich von 0,01 bis 1 Gew.-% aufweisen.

Kapseln für die Inhalation

Der Wirkstoff gemäß der allgemeinen Formel I wird in mikronisierter Form (Partikelgröße im wesentlichen zwischen 2 und 6 μM), gegebenfalls unter Zusatz von mikronisierten Trägersubstanzen, etwa Laktose, in Hartgelatinekapseln gefüllt. Zur Inhalation dienen übliche Geräte für die Pulverinhalation. In jede Kapsel werden z.B. zwischen 0,2 und 20 mg Wirkstoff und 0 bis 40 mg Laktose eingefüllt.

Inhalationsaerosol

| | |
|---|---|
| Wirkstoff gemäß allgemeiner Formel I | 1 Teil |
| Sojalecithin | 0,2 Teile |
| Treibgasmischung ad 100 Teile | |

Die nachfolgenden Beispiele dienen lediglich der exemplarischen Erläuterung ohne den Gegenstand der Erfindung auf selbige zu beschränken.

**Beispiel 1:** Hydroxymethylen-2,6-dimethylphenylacetonitril (3)

126 g (0,87 mol) 2,6-Dimethylphenylacetonitril werden in 450 mL Toluol gelöst und mit 450 mL Ameisensäureethylester versetzt. Die Mischung wird auf eine Temperaur von -10 °C gekühlt und mit 144 g (1,29 mol) Kalium-*tert.*-butylat portionsweise versetzt, so daß die Temperatur nicht über -5 °C steigt. Man läßt über einen Zeitraum von 30

Minuten (min) bei -5 °C und danach 2 Stunden (h) bei Raumtemperatur nachreagieren. Anschließend wird zweimal mit 500 mL Wasser extrahiert, die wäßrige Phase mit 100 mL konzentrierter Salzsäure sauer gestellt und wieder zweimal mit je 500 mL Methylenchlorid (Dichlormethan) extrahiert. Die organische Phase wird getrocknet und im Vakuum (i. Vak.) eingeengt. Der Rückstand wird aus Petrolether umkristallisiert. Ausbeute: 139 g (92% d. Th.).

**Beispiel 2:** 3-Hydroxy-2-(2,6-dimethylphenyl)-propionitril (4)

58 g (0,33 mol) Hydroxymethylen-2,6-dimethylphenylacetonitril werden in 250 mL Methanol gelöst und mit 37 g (0,59 mol) Natriumcyanoborhydrid versetzt. Nach 2 h werden 250 mL Essigsäure zugetropft, wobei sich die Mischung auf 50 °C erwärmt. Nach dem Abklingen der exothermen Reaktion wird noch 3 h unter Rückfluß erhitzt. Man läßt abkühlen und engt die Mischung im Vakuum ein. Der Rückstand wird mit 500 g Eis versetzt, mit Ammoniak neutralisiert und zweimal mit 500 mL Essigsäureethylester (Essigester) extrahiert. Die vereinigten organischen Extrakte werden mit 100 mL Wasser gewaschen. Nach dem Trocknen wird das Lösungsmittel im Vakuum abgezogen. Der Rückstand wird roh weiterverarbeitet. Man erhält 54,1 g (92% d. Th.) der Titelverbindung in Form eines Öls.

**Beispiel 3:** 3-Hydroxy-2-(2,6-dimethylphenyl)-propylamin (5)

52,4 g (0,3 mol) 3-Hydroxy-2-(2,6-dimethylphenyl)-propionitril werden in 400 mL Methanol gelöst und mit 50 g $NH_3$ versetzt. Die Reaktionsmischung wird unter einem Druck von 70 bar sowie bei einer Reaktionstemperatur von 70 °C in Gegenwart von 60 g Raney-Nickel hydriert. Nach 4 h läßt man abkühlen, filtriert über Kieselgel und zieht das Lösungmittel im Vakuum ab. Der Rückstand wird im Hochvakuum destilliert. Ausbeute: 40,6 g (76%), Sdp.: 135-140 °C (0,02 mbar); Schmp.: des Hydrochlorids: 166 °C.

**Beispiel 4.1:** (-)-3-Hydroxy-2-(2,6-dimethylphenyl)-propylamin-(S)-tartrat (5a)

74,5 g (0,41 mol) 3-Hydroxy-2-(2,6-dimethylphenyl)-propylamin werden in 745 mL Methanol gelöst und mit 62,4 g (0,41 mol) S-(-)-Weinsäure versetzt. Nach 15 min. werden die ausgefallenen Kristalle abgesaugt und je einmal mit 50 mL Methanol und 50 mL Diethylether (Ether) gewaschen. Anschließend wird dreimal aus Methanol umkristallisiert. Ausbeute: 45,3 g (34%), Schmp.: 181 °C, $[\alpha]_D^{25}$ = (-) 21,8° (c=1 in Methanol).

Analog zu Beispiel 4.1 wird dargestellt:

Beispiel 4.2:

(+)-3-Hydroxy-2-(2,6-dimethylphenyl)-propylamin-(R)-tartrat: Schmp.: 181 °C, $[\alpha]_D^{25}$ = (+) 22,3° (c=1 in Methanol).

**Beispiel 5.1:** N-*tert.*-Butoxycarbonyl-3-hydroxy-2-(2,6-dimethylphenyl)-propylamin (6)

14 g (78 mmol) 3-Hydroxy-2-(2,6-dimethylphenyl)-propylamin werden in 140 mL Essigester gelöst und bei -10 °C mit 20,5 g (94 mmol) Pyrokohlensäure-di-*tert.*butylester versetzt. Man läßt 1 h bei 0 °C und 2 h bei RT nachreagieren, zieht das Lösungsmittel im Vakuum ab und versetzt mit 100 mL 90%iger Essigsäure. Nach 1 h bei 50 °C wird im Vakuum eingeengt, der Rückstand in 200 mL Essigester aufgenommen und zweimal mit 100 mL 10%iger Ammoniaklösung gewaschen. Die organische Phase wird getrocknet und das Lösungsmittel i. Vak. abgezogen.
Ausbeute: 19,3 g (89%), Öl.

Analog zu Beispiel 5.1 werden dargestellt:

Beispiel 5.2:

(+)-N-*tert.*-Butoxycarbonyl-3-hydroxy-2-(2,6-dimethylphenyl)-propylamin, Schmp.: 103 °C.

Beispiel 5.3:

(-)-N-*tert.*-Butoxycarbonyl-3-hydroxy-2-(2,6-dimethylphenyl)-propylamin, , Schmp.: 103 °C.

**Beispiel 6.1:** N-*tert.*-Butoxycarbonyl-3-(2,6-difluorphenyl)methoxy-2-(2,6-dimethylphenyl)-propylamin (7)

19 g (68 mmol) N-*tert.*-Butoxycarbonyl-3-hydroxy-2-(2,6-dimethylphenyl)-propylamin werden in 150 mL $CH_2Cl_2$ gelöst, mit 100 mL 50%-iger Natronlauge, 3 g Tetrabutylammoniumhydrogensulfat und 16,5 g (80 mmol) 2,6-Difluorbenzylbromid versetzt. Man rührt 3 h bei Raumtemperatur, trennt die organische Phase ab und extrahiert die wässerige Phase einmal mit 100 mL Dichlormethan. Die vereinigten organischen Phasen werden einmal mit 50 mL 2 N Salzsäure gewaschen, getrocknet und das Lösungsmittel im Vakuum abgezogen. Der Rückstand wird aus Diisopropylether kristallisiert. Ausbeute: 23,5 g (85,1 % d. Th.), Schmp.: 100 °C.

Analog zu Beispiel 6.1 werden hergestellt:

Beispiel 6.2:

(+)-N-*tert.*-Butoxycarbonyl-3-(2,6-difluorphenyl)methoxy-2-(2,6-dimethylphenyl)-propylamin, Schmp.: 104 °C.

Beispiel 6.3:

(-)-N-*tert.*-Butoxycarbonyl-3-(2,6-difluorphenyl)methoxy-2-(2,6-dimethylphenyl)-propylamin, Schmp.: 104 °C.

Beispiel 6.4:

N-*tert.*-Butoxycarbonyl-3-(2-fluorphenyl)methoxy-2-(2,6-dimethylphenyl)-propylamin, Öl.

Beispiel 6.5:

(+)-N-*tert.*-Butoxycarbonyl-3-(2-fluorphenyl)methoxy-2-(2,6-dimethylphenyl)-propylamin, Schmp.: 102 °C.

Beispiel 6.6:

(-)-N-*tert.*-Butoxycarbonyl-3-(2-fluorphenyl)methoxy-2-(2,6-dimethylphenyl)-propylamin, Schmp.: 102 C.

Beispiel 6.7:

N-*tert.*-Butoxycarbonyl-3-(2-chlor-6-fluorphenyl)methoxy-2-(2,6-dimethylphenyl)-propylamin, Öl.

Beispiel 6.8:

N-*tert.*-Butoxycarbonyl-3-(2,6-dimethylphenyl)methoxy-2-(2,6-dimethylphenyl)-propylamin, Öl.

**Beispiel 7.1:** 3-(2,6-Difluorphenyl)methoxy-2-(2,6-dimethylphenyl)-propylaminhydrochlorid (8)

In eine Lösung aus 23 g (57 mmol) N-tert.-Butoxycarbonyl-3-(2,6-difluorphenyl)methoxy-2-(2,6-dimethylphenyl)-propylamin in 23 mL Essigester wird für 3 min ein kräftiger Strom von HCl-Gas eingeleitet. Man läßt 30 min nachreagieren, zieht das Lösungsmittel im Vakuum ab und nimmt den Rückstand in 30 mL Toluol auf. Das Lösungsmittel wird erneut im Vakuum abgezogen und der Rückstand aus Ether kristallisiert. Ausbeute: 18,5 g (95%), Schmp.: 123 °C.

Analog zu Beispiel 7.1 werden dargestellt:

Beispiel 7.2:

(+)-3-(2,6-Difluorphenyl)methoxy-2-(2,6-dimethylphenyl)-propylamin-hydrochlorid; Schmp.: 154 °C, $[\alpha]_D^{25}$ = (+) 9,0° (c=1 in Methanol).

Beispiel 7.3:

(-)-3-(2,6-Difluorphenyl)methoxy-2-(2,6-dimethylphenyl)-propylamin-hydrochlorid; Schmp.: 155 °C, $[\alpha]_D^{25}$ =

(-) 8,9° (c=1 in Methanol).

<u>Beispiel 7.4:</u>

3-(2-Fluorphenyl)methoxy-2-(2,6-dimethylphenyl)-propylamin-tartrat; Schmp.: 193 °C.

<u>Beispiel 7.5:</u>

(+)-3-(2-Fluorphenyl)methoxy-2-(2,6-dimethylphenyl)-propylamin-hydrochlorid; Schmp.: 145 °C, $[\alpha]_D^{25}$ = (+) 4,7° (c=1 in Methanol).

<u>Beispiel 7.6:</u>

(-)-3-(2-Fluorphenyl)methoxy-2-(2,6-dimethylphenyl)-propylamin-hydrochlorid; Schmp.: 144 °C, $[\alpha]_D^{25}$ = (-) 4,5° (c=1 in Methanol).

<u>Beispiel 7.7:</u>

3-(2-Chlor-6-fluorphenyl)methoxy-2-(2,6-dimethylphenyl)-propylamin-hydrochlorid; Schmp.: 128 °C.

<u>Beispiel 7.8:</u>

3-(2,6-Dimethylphenyl)methoxy-2-(2,6-dimethylphenyl)-propylamin-hydrochlorid; Schmp.: 154 °C.

**Beispiel 8.1:** <u>N-Allyl-3-(2,6-difluorphenyl)methoxy-2-(2,6-dimethylphenyl)-propylamin-hydrobromid (1)</u>

1 g (3,3 mmol) 3-(2,6-Difluorphenyl)methoxy-2-(2,6-dimethylphenyl)-propylamin werden in 10 mL Tetrahydrofuran (THF) gelöst und mit 0,4 g (3,3 mmol) Allylbromid und 1,2 g $K_2CO_3$ 16 h bei Raumtemperatur gerührt. Anschließend wird das Lösungsmittel im Vakuum abgezogen, der Rückstand mit 50 mL Wasser versetzt und zweimal mit je 30 mL Essigester extrahiert. Die organische Phase wird getrocknet, i. Vak. eingeengt und der Rückstand an Kieselgel chromatographiert (Methanol/Dichlormethan = 2 : 98). Die geeigneten Fraktionen werden eingeengt, der Rückstand in Ether gelöst und mit HBr/Eisessig das Hydrobromid gefällt. Ausbeute: 1,0 g (71%), Schmp.: 105 °C.
Analog zu Beispiel 8.1 werden dargestellt (gegebenenfalls unter Verwendung von DMF als Lösungsmittel):

<u>Beispiel 8.2:</u>

N-Propyl-3-(2,6-difluorphenyl)methoxy-2-(2,6-dimethylphenyl)-propylaminhydrochlorid; Schmp.: 142 °C.

<u>Beispiel 8.3:</u>

N,N-Dipropyl-3-(2,6-difluorphenyl)methoxy-2-(2,6-dimethylphenyl)-propylaminhydrobromid; Schmp.: 143 °C.

<u>Beispiel 8.4:</u>

N-Isopropyl-3-(2,6-difluorphenyl)methoxy-2-(2,6-dimethylphenyl)-propylaminhydrochlorid; Schmp.: 138 °C.

<u>Beispiel 8.5:</u>

N-Isobutyl-3-(2,6-difluorphenyl)methoxy-2-(2,6-dimethylphenyl)-propylaminhydrochlorid; Schmp.: 119 °C.

<u>Beispiel 8.6:</u>

N-Cyclopentyl-3-(2,6-difluorphenyl)methoxy-2-(2,6-dimethylphenyl)-propylaminhydrochlorid; Schmp.: 130 °C.

Beispiel 8.7:

N,N-Dimethyl-3-(2,6-difluorphenyl)methoxy-2-(2,6-dimethylphenyl)-propylaminhydrochlorid; Schmp.: 144 °C.

Beispiel 8.8:

N-Cyclopropylmethyl-3-(2,6-difluorphenyl)methoxy-2-(2,6-dimethylphenyl)-propylamin-hydrochlorid; Schmp.: 145 °C.

Beispiel 8.9:

N-Propargyl-3-(2,6-difluorphenyl)methoxy-2-(2,6-dimethylphenyl)-propylaminhydrochlorid; Schmp.: 124 °C.

Beispiel 8.10:

(+)-N-Propargyl-3-(2-fluorphenyl)methoxy-2-(2,6-dimethylphenyl)-propylaminhydrochlorid; Schmp.: 112 °C, $[\bar{\alpha}]_D^{25}$ = (+) 6,2° (c=1 in Methanol).

Beispiel 8.11:

(-)-N-Propargyl-3-(2-fluorphenyl)methoxy-2-(2,6-dimethylphenyl)-propylaminhydrochlorid; Schmp.: 112 °C, $[\alpha]_D^{25}$= (-) 6,3° (c=1 in Methanol).

Beispiel 8.12:

(-)-N-Propargyl-3-(2,6-difluorphenyl)methoxy-2-(2,6-dimethylphenyl)-propylaminhydrochlorid; Schmp.: 145 °C, $[\alpha]_D^{25}$= (-) 2,2° (c=1 in Methanol).

Beispiel 8.13:

N-Methoxyethyl-3-(2,6-difluorphenyl)methoxy-2-(2,6-dimethylphenyl)-propylaminhydrochlorid; Schmp.: 128 °C.

Beispiel 8.14:

N-Propyl-3-(2-fluorphenyl)methoxy-2-(2,6-dimethylphenyl)-propylamin-hydrochlorid; Schmp.: 100 °C.

Beispiel 8.15:

N-Isopropyl-3-(2-fluorphenyl)methoxy-2-(2,6-dimethylphenyl)-propylaminhydrochlorid; Schmp.: 123 °C.

Beispiel 8.16:

N-Cyclopentyl-3-(2-fluorphenyl)methoxy-2-(2,6-dimethylphenyl)-propylaminhydrochlorid; Schmp.: 102 °C.

Beispiel 8.17:

(+)-N-(2-Tetrahydrofurylmethyl)-3-(2,6-difluorphenyl)methoxy-2-(2,6-dimethylphenyl)-propylamin-hydrobromid; Als Elektrophil wurde D-Camphersulfonsäure-Stetrahydrofurfurylester eingesetzt. Schmp.: 149 °C, $[\alpha]_D^{25}$ = (+) 17,8° (c=1 in Methanol).

Beispiel 8.18:

(-)-N-Pentamethylen-3-(2,6-difluorphenyl)methoxy-2-(2,6-dimethylphenyl)-propylamin-hydrochlorid; Als Elektrophil wurde 1,5-Dibrompentan eingesetzt. Schmp.: 180 °C, $[\alpha]_D^{25}$ = (-) 7.6° (c=1 in Methanol).

Beispiel 8.19:

N-Pentamethylen-3-(2,6-difluorphenyl)methoxy-2-(2,6-difluorphenyl)-propylaminhydrochlorid;

**Beispiel 9.1:** N-(2-R-2-Methoxypropyl)-3-(2,6-difluorphenyl)methoxy-2-(2,6-dimethylphenyl)-propylamin-hydrobromid (1)

1,4 g (4,6 mmol) 3-(2,6-Difluorphenyl)methoxy-2-(2,6-dimethylphenyl)-propylamin werden in 20 mL Essigester gelöst und mit 2 g Triethylamin versetzt. Die Mischung wird auf 0 °C gekühlt und 2 g (16 mmol) R-(+)-2-Methoxypropionsäurechlorid in 10 mL Essigester zugetropft. Nach 30 min Nachreaktion wird zweimal mit je 50 mL 2 N Salzsäure gewaschen. Die organische Phase wird getrocknet und im Vakuum eingeengt. Der Rückstand wird in THF aufgenommen, und man gibt 1 g Natriumborhydrid ($NaBH_4$) und 3 mL Bortrifluorid-Etherat ($BF_3$-$Et_2O$) zu. Man läßt 12 h nachreagieren, versetzt mit 5 mL Wasser und anschließend 30 mL 2 N Salzsäure. Das THF wird im Vakuum abgezogen. Anschließend versetzt man mit 30 mL Ethanol und erwärmt für 30 min auf 70°C. Dann wird das Ethanol im Vakuum abgezogen, die wäßrige Phase mit 30 mL Ammoniak-Lösung versetzt und zweimal mit je 30 mL Essigester extrahiert. Die organische Phase wird getrocknet, im Vakuum eingeengt, und der Rückstand an Kieselgel chromatographiert (Methanol/Dichlormethan = 2 : 98). Die geeigneten Fraktionen werden eingeengt und der Rückstand in Ether gelöst. Aus der Lösung wird das Hydrobromid mit HBr/Eisessig gefällt. Ausbeute: 0,9 g (43%), 1:1 Diastereomerengemisch, Schmp.: 154 °C.

Analog zu Beispiel 9.1 werden dargestellt:

Beispiel 9.2:

N-(2-S-2-Methoxypropyl)-3-(2,6-difluorphenyl)methoxy-2-(2,6-dimethylphenyl)-propylamin-hydrobromid; 1:1 Diastereomerengemisch, Schmp.: 151 °C.

Beispiel 9.3:

(-)-N-(2-R-2-Methoxypropyl)-3-(2,6-difluorphenyl)methoxy-2-(2,6-dimethylphenyl)-propylamin-hydrochlorid; Schmp.: 105 °C, $[\alpha]_D^{25}$ = (-) 15,1° (c=1 in Methanol).

Beispiel 9.4:

(-)-N-(2-R-2-Methoxypropyl)-3-(2,6-difluorphenyl)methoxy-2-(2,6-dimethylphenyl)-propylamin-hydrobromid; Schmp.: 94 °C, $[\alpha]_D^{25}$ = (-) 11,1° (c=1 in Methanol).

Beispiel 9.5:

(+)-N-(2-S-2-Methoxypropyl)-3-(2,6-difluorphenyl)methoxy-2-(2,6-dimethylphenyl)-propylamin-hydrobromid; Schmp.: 131 °C, $[\alpha]_D^{25}$ = (+) 12,9° (c=1 in Methanol).

Beispiel 9.6:

(+)-N-(2-S-2-Methoxypropyl)-3-(2,6-difluorphenyl)methoxy-2-(2,6-dimethylphenyl)-propylamin-hydrobromid; Schmp.: 130 °C, $[\alpha]_D^{25}$ = (+) 10,6° (c=1 in Methanol).

Beispiel 9.7:

(-)-N-(2-R-2-Methoxypropyl)-3-(2-fluorphenyl)methoxy-2-(2,6-dimethylphenyl)-propylamin-diglycolat; Schmp.: 105 °C, $[\alpha]_D^{25}$ = (-) 13,5° (c=1 in Methanol).

Beispiel 9.8:

(-)-N-(2-R-2-Methoxypropyl)-3-(2-fluorphenyl)methoxy-2-(2,6-dimethylphenyl)-propylamin-diglycolat; Schmp.: 77 °C, $[\alpha]_D^{25}$ = (-) 10,6° (c=1 in Methanol).

Beispiel 9.9:

(+)-N-(2-S-2-Methoxypropyl)-3-(2-fluorphenyl)methoxy-2-(2,6-dimethylphenyl)-propylamin-diglycolat; Schmp.: 107 °C, $[\alpha]_D^{25}$ = (+) 13,5° (c=1 in Methanol).

Beispiel 9.10:

(+)-N-(2-S-2-Methoxypropyl)-3-(2-fluorphenyl)methoxy-2-(2,6-dimethylphenyl)-propylamin-diglycolat; Schmp.: 106 °C, $[\alpha]_D^{25}$ = (+) 13,4° (c=1 in Methanol).

Beispiel 9.11:

(+)-N-(2-Methoxyethyl)-3-(2,6-difluorphenyl)methoxy-2-(2,6-dimethylphenyl)-propylamin-hydrochlorid; Schmp.: 136 °C, $[\alpha]_D^{25}$ = (+) 2,5° (c=1 in Methanol).

Beispiel 9.12:

(-)-N-(2-Methoxyethyl)-3-(2,6-difluorphenyl)methoxy-2-(2,6-dimethylphenyl)-propylamin-hydrochlorid; Schmp.: 132 °C, $[\alpha]_D^{25}$ = (-) 2,5° (c=1 in Methanol).

Beispiel 9.13:

(+)-N-(2-Methoxyethyl)-3-(2-fluorphenyl)methoxy-2-(2,6-dimethylphenyl)-propylaminhydrochlorid; Schmp.: 77 °C, $[\alpha]_D^{25}$ = (+) 0,8° (c=1 in Methanol).

Beispiel 9.14:

(-)-N-(2-Methoxyethyl)-3-(2-fluorphenyl)methoxy-2-(2,6-dimethylphenyl)-propylaminhydrochlorid; Schmp.: 77 °C, $[\alpha]_D^{25}$ = (-) 1,0° (c=1 in Methanol).

Beispiel 9.15:

N-Methyl-3-(2,6-difluorphenyl)methoxy-2-(2,6-dimethylphenyl)-propylaminhydrochlorid, als Electrophil wurde Ameisensäureethylester eingesetzt; Schmp.: 153 °C.

Beispiel 9.16:

N-Ethyl-3-(2,6-difluorphenyl)methoxy-2-(2,6-dimethylphenyl)-propylaminhydrochlorid, als Electrophil wurde Acetanhydrid eingesetzt; Schmp.: 153 °C.

Beispiel 9.17:

(+)-N-Cyclopropylmethyl-3-(2,6-difluorphenyl)methoxy-2-(2,6-dimethylphenyl)-propylamin-hydrochlorid; Schmp.: 157 °C, $[\alpha]_D^{25}$ = (+) 4,8° (c=1 in Methanol).

Beispiel 9.18:

(-)-N-Cyclopropylmethyl-3-(2,6-difluorphenyl)methoxy-2-(2,6-dimethylphenyl)-propylamin-hydrochlorid; Schmp.: 156 °C, $[\alpha]_D^{25}$ = (-) 4,8° (c=1 in Methanol).

Beispiel 9.19:

N-Cyclopropylmethyl-3-(2-chlor-6-fluorphenyl)methoxy-2-(2,6-dimethylphenyl)-propylamin-hydrochlorid; Schmp.: 154 °C.

Beispiel 9.20:

N-Cyclopropylmethyl-3-(2,6-dimethylphenyl)methoxy-2-(2,6-dimethylphenyl)-propylamin-hydrochlorid;

Schmp.: 142 °C.

Beispiel 9.21:

(+)-N-Cyclopropylmethyl-3-(2-fluorphenyl)methoxy-2-(2,6-dimethylphenyl)-propylamin-hydrobromid; Schmp.: 101 °C, $[\alpha]_D^{25}$ = (+) 1,4° (c=1 in Methanol).

Beispiel 9.22:

(-)-N-Cyclopropylmethyl-3-(2-fluorphenyl)methoxy-2-(2,6-dimethylphenyl)-propylamin-hydrochlorid; Schmp.: 101 °C, $[\alpha]_D^{25}$ = (-) 1,5° (c=1 in Methanol).

Beispiel 9.23:

N-Cyclobutylmethyl-3-(2-fluorphenyl)methoxy-2-(2,6-dimethylphenyl)-propylaminhydrochlorid; Schmp.: 191 °C.

Beispiel 9.24:

(+)-N-Propyl-3-(2,6-difluorphenyl)methoxy-2-(2,6-dimethylphenyl)-propylaminhydrochlorid, als Elektrophil wurde Propionsäureanhydrid eingesetzt; Schmp.: 136 °C, $[\alpha]_D^{25}$ = (+) 2,8° (c=1 in Methanol).

Beispiel 9.25:

(-)-N-Propyl-3-(2,6-difluorphenyl)methoxy-2-(2,6-dimethylphenyl)-propylaminhydrochlorid, als Elektrophil wurde Propionsäureanhydrid eingesetzt; Schmp.: 133 °C, $[\alpha]_D^{25}$ = (-) 2,3° (c=1 in Methanol).

Beispiel 9.26:

(+)-N-Propyl-3-(2-fluorphenyl)methoxy-2-(2,6-dimethylphenyl)-propylamin-diglycolat, als Elektrophil wurde Propionsäureanhydrid eingesetzt; Schmp.: 115 °C, $[\alpha]_D^{25}$ = (+) 2,8° (c=1 in Methanol).

Beispiel 9.27:

(-)-N-Propyl-3-(2-fluorphenyl)methoxy-2-(2,6-dimethylphenyl)-propylamin-diglycolat, als Elektrophil wurde Propionsäureanhydrid eingesetzt; Schmp.: 115 °C, $[\alpha]_D^{25}$ = (-) 2,8° (c=1 in Methanol).

Beispiel 9.28:

(+)-N-Pentyl-3-(2,6-difluorphenyl)methoxy-2-(2,6-dimethylphenyl)-propylaminhydrochlorid, Schmp.: 109 °C, $[\alpha]_D^{25}$ = (+) 3,0° (c=1 in Methanol).

Beispiel 9.29:

(-)-N-Pentyl-3-(2,6-difluorphenyl)methoxy-2-(2,6-dimethylphenyl)-propylaminhydrochlorid; Schmp.: 100 °C, $[\alpha]_D^{25}$ = (-) 3,5° (c=1 in Methanol).

Beispiel 9.30:

(+)-N-Pentyl-3-(2-fluorphenyl)methoxy-2-(2,6-dimethylphenyl)-propylamin-diglycolat; Schmp.: 106 °C, $[\alpha]_D^{25}$ = (+) 1,5° (c=1 in Methanol).

Beispiel 9.31:

(-)-N-Pentyl-3-(2-fluorphenyl)methoxy-2-(2,6-dimethylphenyl)-propylamin-diglycolat; Schmp.: 115 °C, $[\alpha]_D^{25}$= (-) 2,0° (c=1 in Methanol).

**Beispiel 10.1:** N-Benzyl-3-(2,6-difluorphenyl)methoxy-2-(2,6-dimethylphenyl)-propylamin-hydrochlorid (1)

0,6 g (2,0 mmol) 3-(2,6-Difluorphenyl)methoxy-2-(2,6-dimethylphenyl)-propylamin werden mit 0,3 g (2,8 mmol) Benzaldehyd 1 h bei 60 °C gerührt. Anschließend werden 10 mL Ethanol und 0,3 g $NaBH_4$ zugesetzt, und man rührt 30 min. bei 50 °C. Man läßt abkühlen, tropft 10 mL 2 N Salzsäure hinzu und zieht das Ethanol im Vakuum ab. Der Rückstand wird mit 10 mL konz. Ammoniak versetzt, und man extrahiert zweimal mit je 30 mL Essigester. Die organische Phase wird mit Wasser gewaschen, getrocknet und im Vakuum eingeengt. Der Rückstand wird in Ether aufgenommen und das Hydrochlorid wird mit etherischer Salzsäure gefällt. Ausbeute: 0,7 g (81%), Schmp.: 179 °C.

Analog zu Beispiel 10.1 wurden dargestellt:

Beispiel 10.2:

N-Cyclohexyl-3-(2,6-difluorphenyl)methoxy-2-(2,6-dimethylphenyl)-propylaminhydrochlorid; Schmp.: 141 °C.

Beispiel 10.3:

N-(2,2-Dimethylpropyl)-3-(2,6-difluorphenyl)methoxy-2-(2,6-dimethylphenyl)-propylamin-hydrochlorid; Schmp.: 176 °C.

Beispiel 10.4:

N-(2-Furylmethyl)-3-(2,6-difluorphenyl)methoxy-2-(2,6-dimethylphenyl)-propylaminhydrochlorid; Schmp.: 136 °C.

Beispiel 10.6:

(+)-N-Isopropyl-3-(2,6-difluorphenyl)methoxy-2-(2,6-dimethylphenyl)-propylaminhydrochlorid. Bei der Umsetzung mit Aceton wurde das Wasser mit Toluol aceotrop entfernt; Schmp.: 107 °C, $[\alpha]_D^{25}$ = (+) 8,1° (c=1 in Methanol).

Beispiel 10.7:

(+)-N-Isopropyl-3-(2-fluorphenyl)methoxy-2-(2,6-dimethylphenyl)-propylaminhydrobromid. Bei der Umsetzung mit Aceton wurde das Wasser mit Toluol aceotrop entfernt; Schmp.: 111 °C, $[\alpha]_D^{25}$ = (+) 3,9° (c=1 in Methanol).

Beispiel 10.8:

(-)-N-Isopropyl-3-(2,6-difluorphenyl)methoxy-2-(2,6-dimethylphenyl)-propylaminhydrobromid. Bei der Umsetzung mit Aceton wurde das Wasser mit Toluol aceotrop entfernt; Schmp.: 112 °C, $[\alpha]_D^{25}$ = (-) 4,5° (c=1 in Methanol).
Unter Anwendung der vorstehend genannten Methoden (Beispiele 8.1, 9.1 oder 10.1) werden ferner die nachfolgenden Verbindungen zugänglich:

Beispiel 11.1:

(+)-N-Cyclohexylmethyl-3-(2,6-difluorphenyl)methoxy-2-(2,6-dimethylphenyl)-propylamin-hydrobromid; Schmp.: 154 °C, , $[\alpha]_D^{25}$ = (+) 2,2° (c=1 in Methanol).

Beispiel 11.2:

(+)-N-Cyclopentylmethyl-3-(2,6-difluorphenyl)methoxy-2-(2,6-dimethylphenyl)-propylamin-hydrobromid; Schmp.: 147 °C, , $[\alpha]_D^{25}$ = (+) 4,3° (c=1 in Methanol).

Beispiel 11.3:

(-)-N-Cyclobutylmethyl-3-(2,6-difluorphenyl)methoxy-2-(2,6-dimethylphenyl)-propylamin-diglycolat; Schmp.: 138 °C, , $[\alpha]_D^{25}$ = (-) 4,1° (c=1 in Methanol).

Beispiel 11.4:

(-)-N,N-Diallyl-3-(2,6-difluorphenyl)methoxy-2-(2,6-dimethylphenyl)-propylaminhydrobromid; Schmp.: 183 °C, , $[\alpha]_D^{25}$ = (-) 2,8° (c=1 in Methanol).

Beispiel 11.5:

(-)-N,N-Di-(3,3-dimethylallyl)-3-(2,6-difluorphenyl)methoxy-2-(2,6-dimethylphenyl)-propylamin-hydrobromid; Schmp.: 139 °C, , $[\alpha]_D^{25}$ = (-) 5,4° (c=1 in Methanol).

Beispiel 11.6:

(+)-N-(3,3-Dimethylallyl)-3-(2,6-difluorphenyl)methoxy-2-(2,6-dimethylphenyl)-propylamin-hydrobromid; Schmp.: 175 °C, , $[\alpha]_D^{25}$ = (+) 5,0° (c=1 in Methanol).

Beispiel 11.7:

(-)-N-Cyclopentylmethyl-3-(2,6-difluorphenyl)methoxy-2-(2,6-dimethylphenyl)-propylamin-hydrobromid; Schmp.: 147 °C, , $[\alpha]_D^{25}$ = (-) 4,3° (c=1 in Methanol).

Beispiel 11.8:

(+)-N-Cyclobutylmethyl-3-(2,6-difluorphenyl)methoxy-2-(2,6-dimethylphenyl)-propylamin-diglycolat; Schmp.: 138 °C, , $[\alpha]_D^{25}$ = (+) 4,0° (c=1 in Methanol).

Beispiel 11.9:

(+)-N-Allyl-3-(2,6-difluorphenyl)methoxy-2-(2,6-dimethylphenyl)-propylaminhydrobromid; Schmp.: 142 °C, , $[\alpha]_D^{25}$= (+) 4,0° (c=1 in Methanol).

Beispiel 11.10:

(-)-N-Allyl-3-(2,6-difluorphenyl)methoxy-2-(2,6-dimethylphenyl)-propylaminhydrobromid; Schmp.: 142 °C, , $[\alpha]_D^{25}$ = (-) 4,3° (c=1 in Methanol).

Beispiel 11.11:

(+)-N,N-Diallyl-3-(2,6-difluorphenyl)methoxy-2-(2,6-dimethylphenyl)-propylaminhydrobromid; Schmp.: 183 °C, , $[\alpha]_D^{25}$= (+) 2,8° (c=1 in Methanol).

Beispiel 11.12:

(+)-N,N-Di-(3,3-dimethylallyl)-3-(2,6-difluorphenyl)methoxy-2-(2,6-dimethylphenyl)-propylamin-hydrobromid; Schmp.: 139 °C, , $[\alpha]_D^{25}$ = (+) 5,0° (c=1 in Methanol).

Beispiel 11.13:

(-)-N-(3,3-Dimethylallyl)-3-(2,6-difluorphenyl)methoxy-2-(2,6-dimethylphenyl)-propylamin-hydrobromid; Schmp.: 175 °C, , $[\alpha]_D^{25}$ = (-) 6,7° (c=1 in Methanol).

Beispiel 11.14:

(+)-N-(2-Ethylbutyl)-3-(2,6-difluorphenyl)methoxy-2-(2,6-dimethylphenyl)-propylaminhydrochlorid; Schmp.: 144 °C, , $[\alpha]_D^{25}$ = (+) 1,5° (c=1 in Methanol).

Beispiel 11.15:

(-)-N-(5-Norbornen-2-yl-methyl)-3-(2,6-difluorphenyl)methoxy-2-(2,6-dimethylphenyl)-propylamin-hydro-

chlorid; Schmp.: 145 °C, $[\alpha]_D^{25}$ = (-) 0,4° (c=1 in Methanol).

Beispiel 11.16:

(+)-N-Pentyl-3-(2,6-difluorphenyl)methoxy-2-(2,6-dimethylphenyl)-propylaminhydrochlorid; Schmp.: 120 °C, , $[\alpha]_D^{25}$ = (+) 2,9° (c=1 in Methanol).

Beispiel 11.17:

(+)-N-(3-Methoxypropyl)-3-(2,6-difluorphenyl)methoxy-2-(2,6-dimethylphenyl)-propylamin-hydrochlorid; Schmp.: 112 °C, , $[\alpha]_D^{25}$ = (+) 5,0° (c=1 in Methanol).

Beispiel 11.18:

(+)-N-(2-Ethoxyethyl)-3-(2,6-difluorphenyl)methoxy-2-(2,6-dimethylphenyl)-propylamin-hydrochlorid; Schmp.: 123 °C, , $[\alpha]_D^{25}$ = (+) 3,3° (c=1 in Methanol).

Beispiel 11.19:

(-)-N-Cyclohexytmethyl-3-(2,6-difluorphenyl)methoxy-2-(2,6-dimethylphenyl)-propylamin-hydrobromid; Schmp.: 155 °C, , $[\alpha]_D^{25}$ = (-) 4,2° (c=1 in Methanol).

Beispiel 11.20:

(-)-N-(2-Ethylbutyl)-3-(2,6-difluorphenyl)methoxy-2-(2,6-dimethylphenyl)-propylaminhydrochlorid; Schmp.: 144 °C, , $[\alpha]_D^{25}$ = (-) 2,5° (c=1 in Methanol).

Beispiel 11.21:

(+)-N-(2-Methylpropyl)-3-(2,6-difluorphenyl)methoxy-2-(2,6-dimethylphenyl)-propylamin-diglycolat; Schmp.: 126 °C, , $[\alpha]_D^{25}$ = (+) 1,9° (c=1 in Methanol).

Beispiel 11.22:

(-)-N-(2-Methylpropyl)-3-(2,6-difluorphenyl)methoxy-2-(2,6-dimethylphenyl)-propylamin-diglycolat; Schmp.: 126 °C, , $[\alpha]_D^{25}$ = (-) 2,8° (c=1 in Methanol).

Beispiel 11.23:

N-Pentamethylen-3-(2,6-difluorphenyl)methoxy-2-(2,6-dimethoxyphenyl)-propylaminhydrochlorid.

Beispiel 11.24:

(-)-N-Isopropyl-3-(2,6-difluorphenyl)methoxy-2-(2,6-dimethylphenyl)-propylaminhydrochlorid; Schmp.: 114 °C, , $[\alpha]_D^{25}$ = (-) 9,0° (c=1 in Methanol).

Beispiel 11.25:

(-)-N-Pentyl-3-(2,6-difluorphenyl)methoxy-2-(2,6-dimethylphenyl)-propylaminhydrochlorid; Schmp.: 126 °C, , $[\alpha]_D^{25}$ = (-) 4,0° (c=1 in Methanol).

Beispiel 11.26:

(-)-N-(2-Ethoxyethyl)-3-(2,6-difluorphenyl)methoxy-2-(2,6-dimethylphenyl)-propylamin-hydrochlorid; Schmp.: 123 °C, , $[\alpha]_D^{25}$= (-) 4,3° (c=1 in Methanol).

Beispiel 11.27:

(-)-N-(3-Methoxypropyl)-3-(2,6-difluorphenyl)methoxy-2-(2,6-dimethylphenyl)-propylamin-hydrochlorid; Schmp.: 83 °C, , $[\alpha]_D^{25}$ = (-) 6,5° (c=1 in Methanol).

Beispiel 11.28:

(+)-N-Tetramethylen-3-(2,6-difluorphenyl)methoxy-2-(2,6-dimethylphenyl)-propylamin-hydrochlorid; Schmp.: 144 °C, , $[\alpha]_D^{25}$ = (+) 0,3° (c=1 in Methanol).

Beispiel 11.29:

(+)-N-Methyl-N-cyclopropylmethyl-3-(2,6-difluorphenyl)methoxy-2-(2,6-dimethylphenyl)-propylamin-oxalat; Schmp.: 136 °C, , $[\alpha]_D^{25}$ = (+) 3,3° (c=1 in Methanol).

Beispiel 11.30:

(+)-N,N-Dimethyl-3-(2,6-difluorphenyl)methoxy-2-(2,6-dimethylphenyl)-propylaminhydrochlorid; Schmp.: 147 °C, , $[\alpha]_D^{25}$ = (+) 8,3° (c=1 in Methanol).

Beispiel 11.31:

(+)-N,N-Di-(cyclopropylmethyl)-3-(2,6-difluorphenyl)methoxy-2-(2,6-dimethylphenyl)-propylamin-hydrobromid; Schmp.: 181 °C, , $[\alpha]_D^{25}$ = (+) 5,5° (c=1 in Methanol).

Beispiel 11.32:

(-)-N-Methyl-N-cyclopropylmethyl-3-(2,6-difluorphenyl)methoxy-2-(2,6-dimethylphenyl)-propylamin-oxalat; Schmp.: 136 °C, , $[\alpha]_D^{25}$ = (-) 4,4° (c=1 in Methanol).

Beispiel 11.33:

(-)-N,N-Di-(cyclopropylmethyl)-3-(2,6-difluorphenyl)methoxy-2-(2,6-dimethylphenyl)-propylamin-hydrobromid; Schmp.: 181 °C, , $[\alpha]_D^{25}$ = (-) 9,9° (c=1 in Methanol).

Beispiel 11.34:

(-)-N-(4-Fluorbutyl)-3-(2,6-difluorphenyl)methoxy-2-(2,6-dimethylphenyl)-propylaminhydrochlorid; Schmp.: 106 °C, , $[\alpha]_D^{25}$ = (-) 3,3° (c=1 in Methanol).

Beispiel 11.35:

(-)-N-(1-Ethylpropyl)-3-(2,6-difluorphenyl)methoxy-2-(2,6-dimethylphenyl)-propylamin-oxalat; Schmp.: 127 °C, , $[\alpha]_D^{25}$ = (-) 10,8° (c=1 in Methanol).

Beispiel 11.36:

(-)-N-Cyclobutyl-3-(2,6-difluorphenyl)methoxy-2-(2,6-dimethylphenyl)-propylaminhydrochlorid; Schmp.: 107 °C, , $[\alpha]_D^{25}$= (-) 8,7° (c=1 in Methanol).

Beispiel 11.37:

(-)-N-Methyl-N-isopropyl-3-(2,6-difluorphenyl)methoxy-2-(2,6-dimethylphenyl)-propylamin-oxalat; Schmp.: 131 °C, , $[\alpha]_D^{25}$ = (-) 2,5° (c=1 in Methanol).

Beispiel 11.38:

(+)-N-Methyl-N-isopropyl-3-(2,6-difluorphenyl)methoxy-2-(2,6-dimethylphenyl)-propylamin-oxalat; Schmp.:

131 °C, , $[\alpha]_D^{25}$ = (+) 1,4° (c=1 in Methanol).

Beispiel 11.39:

(-)-N-(2-Methylallyl)-3-(2,6-difluorphenyl)methoxy-2-(2,6-dimethylphenyl)-propylaminhydrobromid; Schmp.: 155 °C, , $[\alpha]_D^{25}$ = (-) 1,9° (c=1 in Methanol).

Beispiel 11.40:

(+)-N-(2-Methylallyl)-3-(2,6-difluorphenyl)methoxy-2-(2,6-dimethylphenyl)-propylamin-hydrobromid; Schmp.: 155 °C, , $[\alpha]_D^{25}$ = (+) 1,3° (c=1 in Methanol).

Beispiel 11.41:

(+)-N-Cyclobutyl-3-(2,6-difluorphenyl)methoxy-2-(2,6-dimethylphenyl)-propylaminhydrochlorid; Schmp.: 146 °C, , $[\alpha]_D^{25}$ = (+) 8,1 ° (c=1 in Methanol).

Beispiel 11.42:

(+)-N-(1-Ethylpropyl)-3-(2,6-difluorphenyl)methoxy-2-(2,6-dimethylphenyl)-propylamin-oxalat; Schmp.: 131 °C, , $[\alpha]_D^{25}$ = (+) 11,6° (c=1 in Methanol).

Beispiel 11.43:

(-)-N-(2-Methylallyl)-N-methyl-3-(2,6-difluorphenyl)methoxy-2-(2,6-dimethylphenyl)-propylamin-oxalat; Schmp.: 128 °C, , $[\alpha]_D^{25}$ = (-) 6,7° (c=1 in Methanol).

Beispiel 11.44:

(+)-N-(2-Methylallyl)-N-methyl-3-(2,6-difluorphenyl)methoxy-2-(2,6-dimethylphenyl)-propylamin-oxalat; Schmp.: 128 °C, , $[\alpha]_D^{25}$ = (+) 5,5° (c=1 in Methanol).

Beispiel 11.45:

(+)-N-(1-Methylallyl)-3-(2,6-difluorphenyl)methoxy-2-(2,6-dimethylphenyl)-propylamin-hydrochlorid; Schmp.: 153 °C, , $[\alpha]_D^{25}$ = (+) 4,4° (c=1 in Methanol).

Beispiel 11.47:

(-)-N-(But-2-enyl)-3-(2,6-difluorphenyl)methoxy-2-(2,6-dimethylphenyl)-propylaminhydrochlorid; Schmp.: 119 °C, , $[\alpha]_D^{25}$ = (-) 5,4° (c=1 in Methanol).

Beispiel 11.48:

(+)-N-Allyl-N-methyl-3-(2,6-difluorphenyl)methoxy-2-(2,6-dimethylphenyl)-propylamin-hydrochlorid; Schmp.: 117 °C, , $[\alpha]_D^{25}$ = (+) 3,0° (c=1 in Methanol).

Beispiel 11.49:

(-)-N-(2-Methylallyl)-3-(2,6-difluorphenyl)methoxy-2-(2,6-dimethylphenyl)-propylaminhydrochlorid; Schmp.: 172 °C, , $[\alpha]_D^{25}$ = (-) 1,6° (c=1 in Methanol).

Beispiel 11.50:

(+)-N-(2-Methylallyl)-3-(2,6-difluorphenyl)methoxy-2-(2,6-dimethylphenyl)-propylamin-hydrochlorid; Schmp.: 172 °C, , $[\alpha]_D^{25}$ = (+) 1,5° (c=1 in Methanol).

Beispiel 11.51:

(+)-N-(But-2-enyl)-3-(2,6-difluorphenyl)methoxy-2-(2,6-dimethylphenyl)-propylaminhydrochlorid; Schmp.: 121 °C, , $[\alpha]_D^{25}$ = (+) 4,6° (c=1 in Methanol).

Beispiel 11.52:

N-Cyclopropylmethyl-3-(2,6-difluorphenyl)methoxy-2-(2,4,6-trimethylphenyl)-propylamin-oxalat; Schmp.: 217°C;

Beispiel 11.53:

N-(1-Methyl-1-cyclopropyl-methyl)-3-(2,6-difluorphenyl)methoxy-2-(2,6-dimethylphenyl)-propylamin-hydrochlorid; Schmp.: 130 °C.

Beispiel 11.54:

N-Butyl-3-(2,6-difluorphenyl)methoxy-2-(2,4,6-trimethylphenyl)-propylaminhydrochlorid; Schmp.: 112 °C.

Beispiel 11.55:

(-)-N-Allyl-N-methyl-3-(2,6-difluorphenyl)methoxy-2-(2,6-dimethylphenyl)-propylaminhydrochlorid; Schmp.: 116 °C, , $[\alpha]_D^{25}$ = (-) 3,9° (c=1 in Methanol).

**Beispiel 12.1:** (S)-4,4-Dimethyl-1-(oxiranylmethyl)-piperidin (9):

Die Verbindung wird änalog J. Amer. Chem. Soc. 80; 1958, 1257 durch Umsetzung von (S)-Epichlorhydrin mit 4,4-Dimethylpiperidin hergestellt, wobei die Reaktionstemperatur von 35 °C nicht überschritten werden soll: Sdp.: 101-104°C/15mbar.

Analog zu Beispiel 12.1 werden hergestellt:

Beispiel 12.2:

(S)-3,3-Dimethyl-1-(oxiranylmethyl)-piperidin,

Beispiel 12.3:

(S)-cis-2,6-Dimethyl-1-(oxiranylmethyl)-piperidin (Reaktionstemperatur 60 °C),

Beispiel 12.4:

(S)-2,2,6,6-Tetramethyl-1-(oxiranylmethyl)-piperidin (Reaktionstemperatur 80 °C),

Beispiel 12.5:

(S)-cis/trans-3,5-Dimethyl-1-(oxiranylmethyl)-piperidin,

Beispiel 12.6:

(S)-1-(Oxiranylmethyl)-tropan.

**Beispiel 13.1:** (+)-N-Pentamethylen-3-hydroxy-2-(2,6-dimethylphenyl)-propylamin (10a)

Aus 2.64 g (0.11 Mol) Magnesium und 19.4 g (0.105 Mol) 2-Brom-*m*-xylol in 100 ml abs. Ether wird eine Grignardlösung bereitet, die zur Vervollständigung der Reaktion noch 1 Stunde auf Rückflußtemperatur erhitzt wird. Bei 25 °C wird anschließend ohne Kühlung innerhalb von 45 Minuten eine Lösung von 14.1 g (0.1 Mol) des literaturbekannten (-)-1-(Oxiranylmethyl)-piperidins in 100 ml absolutem Ether zugegeben und das Reaktionsgemisch 1 Stunde

zum Rückfluß erhitzt. Der Ansatz wird anschließend bei Raumtemperatur vorsichtig mit 100 ml gesättigter Ammoniumchloridlösung zersetzt, die Etherphase abgetrennt, die Wasserphase einmal mit ca. 100 ml Ether extrahiert und die vereinigten Etherextrakte nach dem Waschen und Trocknen am Rotationsverdampfer eingeengt. Das erhaltene Rohprodukt von 10a und 10b wird in 8 - 10 g-Portionen über eine Flashsäule (h = 20 cm, Durchmesser 6 cm, Füllung 250 g Kieselgel 0.04 - 0.063 mm) mit ca. 3.5 l Metylenchlorid/Methanol = 95/5 getrennt. Das gewünschte Regioisomer 10a ($R_f$-Wert ca. 0.4, Regioisomer 10b $R_f$-Wert ca. 0.35) wird in 34 % Ausbeute isoliert. $[\alpha]_D^{20}$ = 16.2° (c = 2, Methanol).

Analog zu Beispiel 13.1 werden hergestellt:

Beispiel 13.2:

(-)-N-Pentamethyten-3-hydroxy-2-(2,6-dimethylphenyl)-propylamin

Beispiel 13.3:

R,S-1-N-[2-(2,6-Dimethylphenyl)-3-hydroxy-propyl]-tropan

Beispiel 13.4:

(+)-N-Pentamethylen-3-hydroxy-2-(2-methylphenyl)-propylamin

Beispiel 13.5:

R,S-N-Pentamethylen-3-hydroxy-2-(4-chlorphenyl)-propylamin

Beispiel 13.6:

R,S-N-Pentamethylen-3-hydroxy-2-phenyl-propylamin

Beispiel 13.7:

R,S-N-Pentamethylen-3-hydroxy-2-(1-naphthyl)-propylamin

Beispiel 13.8:

R,S-N-Pentamethylen-3-hydroxy-2-(2-naphthyl)-propylamin

Beispiel 13.9:

(+)-N-(1,5-Dimethylpentamethylen)-3-hydroxy-2-(2,6-dimethylphenyl)-propylamin

Beispiel 13.10:

R,S-N-Hexamethylen-3-hydroxy-2-(4-chlorphenyl)-propylamin

Beispiel 13.11:

(+)-N-(3,3-Dimethylpentamethylen)-3-hydroxy-2-(2,6-dimethylphenyl)-propylamin

Beispiel 13.12:

(+)-N-(2,4-Dimethylpentamethylen)-3-hydroxy-2-(2,6-dimethylphenyl)-propylamin

Beispiel 13.13:

R,S-N-(1,1,5,5-Tetramethylpentamethylen)-3-hydroxy-2-(2,6-dimethylphenyl)-propylamin

Beispiel 13.14:

(+)-1-N-[2-(2,6-Dimethylphenyl)-3-hydroxy-propyl]-tropan

Beispiel 13.15:

R,S-N-Pentamethylen-3-hydroxy-2-(2,3-dimethylphenyl)-propylamin

Beispiel 13.16:

R,S-N-Pentamethylen-3-hydroxy-2-(2,6-diethylphenyl)-propylamin-hydrochlorid, Schmp. 187-189°C;

Beispiel 13.17:

R,S-N-Pentamethylen-3-hydroxy-2-(2,6-dimethylphenyl)-propylamin

Alternativ zu der gemäß Beispiel 13.1 beschriebenen Vorgehensweise kann auch nach folgender Vorgehensweise verfahren werden, die an Hand der Synthese der Verbindung N-Pentamethylen-3-hydroxy-2-(2,6-dimethylphenyl)-propylaminhydrochlorid (Beispiel 13.17) nachfolgend beschrieben wird.

Aus 1.82 g (0.076 Mol) Magnesium und 10 mL (0.076 Mol) 2-Brom-m-xylol in 40 mL abs. Diethylether wird eine Grignardlösung bereitet, die zur Vervollständigung der Reaktion nach ca.90 Minuten zum Rückfluß erhitzt wird. In der Siedehitze werden 6.8 mL Dioxan (0..8 Mol) in 7 mL abs. Diethylether innerhalb von 2 Stunden zugetropft, wobei der Magnesiumbromid-Dioxan-Komplex feinkristallin ausfällt. Nach 20-stündigem Rühren der Suspension bei Raumtemperatur wird unter Rückfluß innerhalb von 30 Minuten eine Lösung von 5.33 g (0.038 Mol) R,S-1-(Oxiranylmethyl)-piperidin in 25 mL abs. Diethylether zugetropft. Der Ansatz wird nach Beendigung der Reaktion mit ca. 75 mL gesättigter Ammoniumchloridlösung zersetzt und extraktiv mit Diethylether aufgearbeitet. Vom verbleibenden Rückstand wird im Wasserstrahlvakuum das bei der Reaktion gebildete Xylol abdestilliert. Das erhaltene Rohprodukt der Regioisomeren (7.04 g) wird entweder in 2.5 g -Portionen an Kieselgel mit Methylenchlorid/Methanol = 95/5 als Eluens aufgereinigt oder alternativ der in Beispiel 13.1. beschriebenen Flash-Chromatographie unterzogen. Man erhält 4.25 g der Verbindung 13.17 (45% Ausbeute).

Schmp.197-198°C (Hydrochlorid);

**Beispiel 14.1:** (+)-N-Pentamethylen-3-(2,6-difluorphenyl)methoxy-2-(2,6-dimethylphenyl)-propylamin-hydrochlorid (1)

24.7 g (0.1 Mol) (+)-N-Pentamethylen-3-hydroxy-2-(2,6-dimethylphenyl)-propylamin werden in 150 ml THF gelöst und mit 13,5 g (0.12 Mol) Kalium-*tert*.-butylat versetzt. Nach 10 Minuten tropft man bei 25 -35 °C unter Kühlung innerhalb von 30 Minuten eine Lösung von 22.8 g (0.11 Mol) 2,6-Difluorbenzylbromid in 100 ml THF zu, rührt 2 Stunden und engt anschließend die Reaktionsmischung bei 50°C am Rotationsverdampfer ein. Der Rückstand wird in 250 ml Methylenchlorid und 150 ml Wasser aufgenommen. Nach dem Abtrennen der Methylenchloridphase wird die Wasserphase mit 150 ml Methylenchlorid extrahiert. Die vereinigten organischen Extrakte werden nach dem Waschen und Trocknen am Rotationsverdampfer bei 40°C eingeengt. Der Rückstand wird in ca. 10 g - Portionen über eine Flashsäule (vgl. Beispiel 13.1) mit Methylenchlorid/Methanol = 95/5 gereinigt. Die so erhaltenen 38 g Base werden in 114 ml Aceton gelöst und bis zu einem pH-Wert von 2 -3 mit etherischer Salzsäure versetzt. Man rührt 30 Minuten, saugt die ausgefallenen Kristalle ab, die aus Aceton umkristallisiert werden, und erhält 16.9 g der Zielverbindung in 41 % Ausbeute. Schmp.: 176 - 178 °C, $[\alpha]_d^{20}$ = 7.5° (c = 2, Methanol).

Analog zu Beispiel 14.1 werden folgende Verbindungen vom Typ 1 hergestellt:

Beispiel 14.2:

(-)-N-Pentamethylen-3-(2,6-difluorphenyl)methoxy-2-(2,6-dimethylphenyl)-propylamin-hydrochlorid, Schmp.: 176 - 178 °C.

Beispiel 14.3:

R,S-N-Pentamethylen-3-(2,6-difluorphenyl)methoxy-2-(2,6-dimethylphenyl)-propylamin-hydrochlorid, Schmp.: 158 - 160 °C.

Beispiel 14.4:

(+)-N-Pentamethylen-3-(2-fluorphenyl)methoxy-2-(2,6-dimethylphenyl)-propylaminhydrochlorid, Schmp.: 126 - 128 °C.

Beispiel 14.5:

(-)-N-Pentamethylen-3-(2-fluorphenyl)methoxy-2-(2,6-dimethylphenyl)-propylaminhydrochlorid, Schmp.: 128 - 130 °C.

Beispiel 14:6:

(+)-N-Pentamethylen-3-(4-chlorphenyl)methoxy-2-(2',6-dimethylphenyl)-propylaminhydrochlorid, Schmp.: 103 -105 °C.

Beispiel 14.7:

(-)-N-Pentamethylen-3-(4-chlorphenyl)methoxy-2-(2,6-dimethylphenyl)-propylaminhydrochlorid, Schmp.: 118 - 120 °C.

Beispiel 14.8:

R,S-N-Pentamethylen-3-(4-chlorphenyl)methoxy-2-(2,6-dimethylphenyl)-propylaminhydrochlorid, Schmp.: 100 - 101 °C.

Beispiel 14.9:

(+)-N-Pentamethylen-3-phenylmethoxy-2-(2,6-dimethylphenyl)-propylaminhydrochlorid, Schmp.: 144 - 146 °C.

Beispiel 14.10:

(+)-N-Pentamethylen-3-(2,6-dichlorphenyl)methoxy-2-(2,6-dimethylphenyl)-propylamin-hydrochlorid, Öl

Beispiel 14.11:

(-)-N-Pentamethylen-3-(2,6-dichlorphenyl)methoxy-2-(2,6-dimethylphenyl)-propylamin-hydrochlorid, Schmp.: 70 - 73 °C.

Beispiel 14.12:

R,S-N-Pentamethylen-3-(2,6-dichlorphenyl)methoxy-2-(2,6-dimethylphenyl)-propylamin-hydrochlorid, Schmp.: 131 - 133 °C.

Beispiel 14.13:

(+)-N-Pentamethylen-3-(4-bromphenyl)methoxy-2-(2,6-dimethylphenyl)-propylaminhydrochlorid, Schmp.: 110 - 112 °C.

Beispiel 14.14:

(-)-N-Pentamethylen-3-(4-bromphenyl)methoxy-2-(2,6-dimethylphenyl)-propylaminhydrochlorid, Schmp.: 124 - 127 °C.

Beispiel 14.15:

R,S-N-Pentamethylen-3-(4-bromphenyl)methoxy-2-(2,6-dimethylphenyl)-propylaminhydrochlorid, Schmp.: 127 - 129 °C.

Beispiel 14.16:

(+)-N-Pentamethylen-3-(2,6-dimethylphenyl)methoxy-2-(2,6-dimethylphenyl)-propylamin-hydrochlorid, Schmp.: 164 - 165 °C.

Beispiel 14.17:

(-)-N-Pentamethylen-3-(2,6-dimethylphenyl)methoxy-2-(2,6-dimethylphenyl)-propylamin-hydrochlorid, Schmp.: 162 -164 °C.

Beispiel 14.18:

R,S-N-Pentamethylen-3-(2,6-dimethylphenyl)methoxy-2-(2,6-dimethylphenyl)-propylamin-hydrochlorid, Schmp.: 137 - 139 °C.

Beispiel 14.19:

(+)-N-(1,5-Dimethylpentamethylen)-3-(2,6-difluorphenyl)methoxy-2-(2,6-dimethylphenyl)-propylamin-hydrochlorid, Schmp.: 85 - 88 °C.

Beispiel 14.20:

R,S-N-(1,5-Dimethylpentamethylen)-3-(2,6-difluorphenyl)methoxy-2-(2,6-dimethylphenyl)-propylamin-hydrochlorid, Schmp.: 146 - 147 °C.

Beispiel 14.21:

R,S-N-(1,5-Dimethylpentamethylen)-3-(2-fluorphenyl)methoxy-2-(2,6-dimethylphenyl)-propylamin-hydrochlorid, Öl.

Beispiel 14.22:

R,S-N-(1,1,5,5-Tetramethylpentamethylen)-3-(2,6-difluorphenyl)methoxy-2-(2,6-dimethylphenyl)-propylamin-hydrochlorid, Öl.

Beispiel 14.23:

R,S-1-N-[2-(2,6-Dimethylphenyl)-3-(2-fluorphenyl)methoxy-propyl]-tropan, Öl.

Beispiel 14.24:

R,S-1-N-[2-(2,6-Dimethylphenyl)-3-(2,6-difluorphenyl)methoxy-propyl]-tropan, Öl

Beispiel 14.25:

(+)-N-(3,3-Dimethylpentamethylen)-3-(2-fluorphenyl)methoxy-2-(2,6-dimethylphenyl)-propylamin-hydrochlorid, Schmp.: 160 - 161 °C.

Beispiel 14.26:

(+)-N-(3,3-Dimethylpentamethylen)-3-(2,6-difluorphenyl)methoxy-2-(2,6-dimethylphenyl)-propylamin-hydrochlorid, Schmp.: 180 - 182°C.

Beispiel 14.27:

(+)-N-(2,4-Dimethylpentamethylen)-3-(2,6-difluorphenyl)methoxy-2-(2,6-dimethylphenyl)-propylamin-hydrochlorid, E/Z 1:1, Öl.

Beispiel 14.56:

(S)-N-Pentamethylen-3-[3,5-di(trifluormethyl)phenyl]methoxy-2-(2.6-dimethylphenyl)-propylamin-hydrochlorid, Schmp.: 158°C.

Beispiel 14.59:

R,S-N-Pentamethylen-3-(2,6-difluorphenyl)methoxy-2-(2,6-diethylphenyl)-propylamin, Öl.

Beispiel 14.60:

(S)-N-Pentamethylen-2-(2,6-dimethylphenyl)-3-[3-(2,6-difluorphenyl)propoxylpropylamin-hydrochlorid; Schmp.: 189-190°C; es wird Natriumhydrid als Säurefänger verwendet;

**Beispiel 15.1:** R,S-N-Pentamethylen-3-(2-fluorphenoxy)-2-(2,6-dimethylphenyl)-propylamin-hydrochlorid (1) :

Nach Mitsunobu werden 2.5 g (0.01 Mol) R,S-N-Pentamethylen-2-(2,6-dimethylphenyl)-3-hydroxy-propylamin, 0.9 ml (0.01 Mol) 2-Fluorphenol und 2,6 g (0.01 Mol) Triphenylphosphin in 30 ml absolutem THF vorgelegt. Bei Raumtemperatur tropft man eine Lösung von 1.6 ml (0.01 Mol) Azodicarbonsäurediethylester in ca. 5 ml abs. THF zu. Nach Rühren über Nacht wird das Reaktionsgemisch 3 Stunden bei 60 °C gehalten, das THF abdestilliert, der Rückstand in Ether suspendiert und mit 2 N Salzsäure sauer gestellt. Nach dem Abtrennen des Ethers wird die wäßrige Phase alkalisch gestellt, mit Methylenchlorid extrahiert und der Rückstand mittels Flash-Chromatographie (Eluens Essigester / Cyclohexan = 25/75) gereinigt. Die erhaltenen 1.4 g farbloses Öl werden in wenig Aceton gelöst und mit etherischer Salzsäure in das Hydrochlorid überführt. Man erhält 0.9 g weiße Kristalle vom Schmp.: 194 - 196 °C.

Analog zu Beispiel 15.1 wird folgende Verbindung hergestellt:

Beispiel 15.2:

R,S-N-Pentamethylen-3-(2,6-difluorphenoxy)-2-(2,6-dimethylphenyl)-propylaminhydrochlorid, Schmp.: 152 -154 °C.

**Beispiel 16.1:** R,S-N-Pentamethylen-2-(2,6-dimethylphenyl)-3-(2-fluorphenylethoxy)-propylamin-hydrochlorid

2.5 g (0.01 Mol) R,S-N-Pentamethylen-2-(2,6-dimethylphenyl)-3-hydroxy-propylamin und 0.6 g (0.01 Mol) gepulvertes Kaliumhydroxid werden 15 Minuten in DMSO (Dimethylsulfoxid) gerührt, dann mit 1.2 g (0.01 Mol) o-Fluorphenylacetylen versetzt und anschließend noch 4 Stunden bei 70 °C gerührt. Das Reaktionsgemisch wird mit Wasser versetzt, mit Methylenchlorid extrahiert und das Solvens nach dem Waschen und Trocknen abgezogen. Der ölige Rückstand wird mittels Flash-Chromatographie (Fließmittel: 1 l Essigester/Cyclohexan = 25/75) gereinigt und das entstandene Reppe-Produkt (2.6 g Z/E-Gemisch) ohne weitere Reinigung mit 0.5 g Pd/$BaSO_4$ in 30 ml Methanol bei 5 bar und bei Raumtemperatur in 3.5 Stunden hydriert. Das Reaktionsgemisch wird filtriert, das Solvens abgezogen und der Rückstand mittels Flash-Chromatographie (Fließmittel: Essigester/Cyclohexan = 1/1) gereinigt. Der Rückstand von 0.4 g wird mit Aceton/etherische Salzsäure in das Hydrochlorid überführt und durch Verreiben mit Ether kristallisiert. Man erhält 0.3 g farblose Kristalle vom Schmp.: 123 - 124 °C.

**Patentansprüche**

1. Verbindungen der allgemeinen Formel **1**

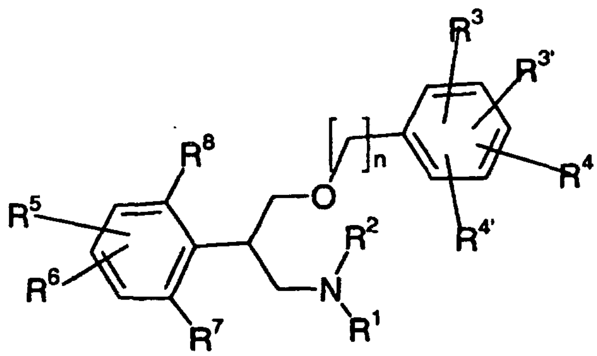

1

worin

| | |
|---|---|
| $R^1$ und $R^2$ | unabhängig voneinander Wasserstoff, $C_1$-$C_6$-Alkyl, Benzyl, Furylmethyl, Cycloalkyl, Cycloalkylmethyl, $C_2$-$C_6$-Alkenyl, $C_2$-$C_6$-Alkinyl, $C_1$-$C_6$-Alkoxy-$(CH_2)_l$-, $C_3$-$C_8$-Cycloalkoxy-$(CH_2)_m$-, und l eine ganze Zahl 1, 2, 3 oder 4, und m eine ganze Zahl 0, 1, 2, 3 oder 4, oder |
| $R^1$ und $R^2$ | zusammen mit dem Stickstoffatom einen 5-, 6- oder 7-gliedrigen heterocyclischen Ring bilden, der gegebenenfalls mit 1-4 Methylgruppen oder einer Dimethylengruppe substituiert sein kann; |
| n | eine ganze Zahl 0, 1, 2 oder 3; |
| $R^3$, $R^4$ und $R^{3'}$ | unabhängig voneinander Wasserstoff, Fluor, Chlor, Brom, Hydroxy, Methyl, Ethyl, Methoxy oder $CF_3$; |
| $R^{4'}$ | Wasserstoff; |
| $R^5$ und $R^6$ | unabhängig voneinander Wasserstoff, Fluor, Chlor, Brom, Methyl oder Ethyl oder |
| $R^5$ und $R^6$ | benachbart einen ankondensierten aromatischen Ring; |
| $R^7$ und $R^8$ | unabhängig von einander Methyl, Ethyl, Methoxy oder Fluor |

bedeuten können, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**2.** Verbindungen der allgemeinen Formel 1 gemäß Anspruch 1, worin

| | |
|---|---|
| $R^1$ und $R^2$ | unabhängig voneinander Wasserstoff, $C_1$-$C_6$-Alkyl, Benzyl, Furylmethyl, Cycloalkyl, Cycloalkyl-methyl, $C_2$-$C_6$-Alkenyl, $C_2$-$C_6$-Alkinyl, $C_1$-$C_6$-Alkoxy-$(CH_2)_l$-, $C_3$-$C_8$-Cycloalkoxy-$(CH_2)_m$-, und l eine ganze Zahl 1, 2, 3 oder 4, und m eine ganze Zahl 0, 1, 2, 3 oder 4, oder |
| $R^1$ und $R^2$ | zusammen mit dem Stickstoffatom einen 5-, 6- oder 7-gliedrigen heterocyclischen Ring bilden, der gegebenenfalls mit 1-4 Methylgruppen oder einer Dimethylengruppe substituiert sein kann; |
| n | eine ganze Zahl 0, 1, 2 oder 3; |
| $R^3$ und $R^4$ | unabhängig voneinander Wasserstoff, Fluor, Chlor, Brom, Hydroxy, Methyl, Ethyl, Methoxy oder $CF_3$; |
| $R^{3'}$ und $R^{4'}$ | Wasserstoff; |
| $R^5$ und $R^6$ | unabhängig voneinander Wasserstoff, Fluor, Chlor, Brom , Methyl, oder Ethyl; |
| $R^7$ und $R^8$ | unabhängig von einander Methyl, Ethyl, Methoxy oder Fluor |

bedeuten können, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**3.** Verbindungen der allgemeinen Formel 1 gemäß einem der Ansprüche 1 oder 2, worin

$R^1$ und $R^2$ unabhängig von einander Wasserstoff, $C_1$-$C_4$-Alkyl, Benzyl, Furylmethyl, Cycloalkyl, Cycloalkyl-methyl, $C_2$-$C_4$-Alkenyl, $C_2$-$C_4$-Alkinyl, $C_1$-$C_4$-Alkoxy-$(CH_2)_l$-, $C_3$-$C_6$-Cycloalkoxy-$(CH_2)_m$-
und
l eine ganze Zahl 1, 2 oder 3,
und
m eine ganze Zahl 1, 2 oder 3,
oder
$R^1$ und $R^2$ zusammen mit dem Stickstoffatom einen 5- oder 6- gliedrigen heterocyclischen Ring bilden, der gegebenenfalls mit 1, 2 öder 3 Methylgruppen oder einer Dimethylengruppe substituiert sein kann;
n eine ganze Zahl 0, 1, 2 oder 3;
$R^3$ Fluor, Chlor oder Methyl;
$R^4$ Wasserstoff, Fluor, Chlor oder Methyl;
$R^{3'}$ und $R^{4'}$ Wasserstoff;
$R^5$ und $R^6$ unabhängig voneinander Wasserstoff oder Methyl;
$R^7$ und $R^8$ unabhängig voneinander Methyl, Ethyl oder Methoxy

bedeuten können, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**4.** Verbindungen der allgemeinen Formel <u>1</u> gemäß einem der Ansprüche 1 bis 3, worin

$R^1$ und $R^2$ unabhängig voneinander Wasserstoff, Methyl, Ethyl, Propyl, Butyl, Benzyl, Furylmethyl, Cyclopentyl, Cyclohexyl, Cyclopropylmethyl, Cyclobutylmethyl, Cyclopentylmethyl, Cyclohexylmethyl, $C_2$-$C_4$-Alkenyl, vorzugsweise Allyl, $C_2$-$C_4$-Alkinyl, vorzugsweise Propargyl, $C_1$-$C_4$-Alkoxy-$(CH_2)_l$-, $C_3$-$C_6$-Cycloalkoxy-$(CH_2)_m$- ,
und
l eine ganze Zahl 1, 2 oder 3
und
m eine ganze Zahl 1, 2 oder 3,
oder
$R^1$ und $R^2$ zusammen mit dem Stickstoffatom einen 5- oder 6-gliedrigen heterocyclischen Ring bilden, der gegebenenfalls mit 1 oder 2 Methylgruppen oder einer Dimethylengruppe substituiert sein kann;
n 1;
$R^3$ *ortho*-Fluor, *ortho*-Chlor oder *ortho*-Methyl;
$R^4$ Wasserstoff, *ortho*-Fluor, *ortho*-Chlor oder *ortho*-Methyl;
$R^{3'}$ und $R^{4'}$ Wasserstoff;
$R^5$ und $R^6$ Wasserstoff;
$R^7$ und $R^8$ gleich oder verschieden, Methyl oder Ethyl,

bedeuten können, gegebenenfalls in Form ihrer Racemate, ihrer Enantiomere, ihrer Diastereomere, Tautomere und ihrer Gemische, sowie gegebenenfalls ihrer pharmakologisch unbedenklichen Säureadditionssalze.

**5.** Pharmazeutische Zubereitung **gekennzeichnet durch** einen Gehalt an einer der Verbindungen nach einem der Ansprüche 1 bis 4 und deren Säureadditionssalze neben üblichen Hilfs- und Trägerstoffen.

**6.** Pharmazeutische Zubereitung nach Anspruch 5, **dadurch gekennzeichnet, daß** sie als Infusionslösung formuliert ist.

**7.** Verwendung von Verbindungen nach einem der Ansprüche1 bis 4 zur Herstellung eines Arzneimittels zur therapeutischen Behandlung von durch Übererregung bedingten Funktionsstörungen.

**8.** Verwendung von Verbindungen nach einem der Ansprüche 1 bis 4 zur Herstellung eines Arzneimittels zur therapeutischen Behandlung von Arrhythmien, Spasmen, kardiale und Gehirnischämien, Schmerzen sowie neurodegenerative Erkrankungen verschiedener Genese.

**9.** Verwendung nach Anspruch 7 oder 8 zur Herstellung eines Arzneimittels zur therapeutischen Behandlung von Epilepsie, Hypoglykämie, Hypoxie, Anoxie, Gehirntrauma, Gehimoedem, Gehirn-Schlaganfall, perinatale Asphyxie, Degenerationen des Cerebellums, amyotrope laterale Sklerose, Morbus Huntington, Morbus Alzheimer, Morbus Parkinson, Zyklophrenie, Hypotonie, Herzinfakt, Herzrhythmusstörungen, Angina pectoris, chronischem Schmerz, neuropathischer Schmerz sowie Lokalanaesthesie.

**10.** Verfahren zur Herstellung von Verbindungen der allgemeinen Formel 1,

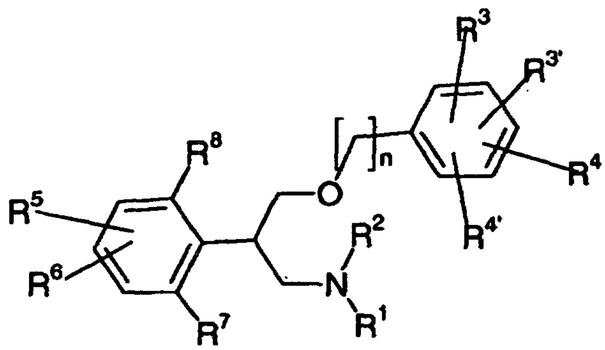

1

worin die Reste $R^1$, $R^2$, $R^3$, $R^{3'}$, $R^4$, $R^{4'}$, $R^5$, $R^6$, $R^7$, $R^8$ und n die in den Ansprüchen 1 bis 4 genannten Bedeutungen haben können,
**dadurch gekennzeichnet, daß** man ein Benzylcyanid der allgemeinen Formel 2

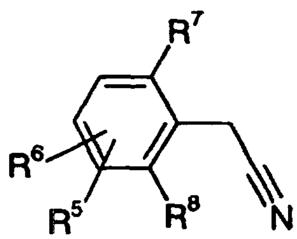

2

worin die Reste $R^5$ bis $R^7$ die vorstehend genannten Bedeutungen haben können, deprotoniert, mit einem Formylierungsmittel umsetzt, die so erhaltene Formylverbindung der allgemeinen Formel 3

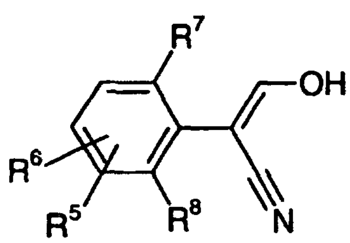

3

und den entsprechenden Alkohol reduziert und das Reaktionsprodukt 4

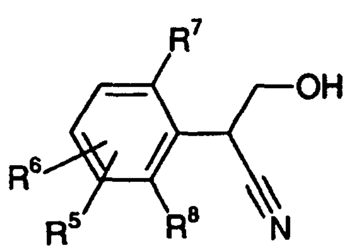

4

zu dem entsprechenden Amin der allgemeinen Formel **5** reduziert

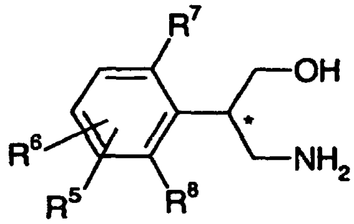

**5**

dieses gegebenenfalls mit an sich aus dem Stand der Technik bekannten Methoden mit Äpfelsäure, Weinsäure, Mandelsäure oder Campfersulfonsäure, worunter Weinsäure besonders bevorzugt ist, in seine Enantiomeren der allgemeinen Formel **5a** oder **5b** auftrennt

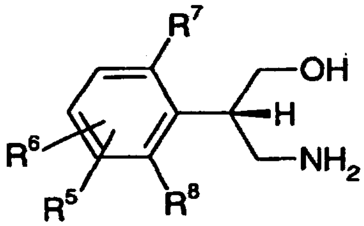

**5a**

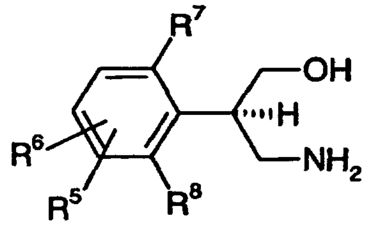

**5b**

oder die Enantiomerenmischung des Amins der allgemeinen Formel 5 mit einem geeigneten Reagens vom Typ X-SG, worin X eine gegen einen Aminstickstoff substituierbare Austrittsgruppe und SG eine für ein primäres Amin geeignete Schutzgruppe bedeutet, in das geschützte Amin der allgemeinen Formel 6 überführt

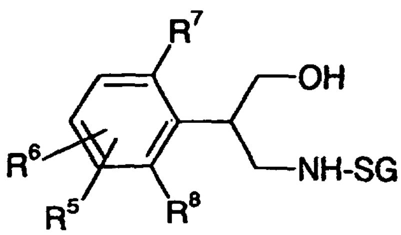

**6**

und das so geschützte Amin mit einem geeigneten Phenylalkylderivat, welches gegebenenfalls mit den Substituenten $R^3$, $R^{3'}$, $R^4$ und $R^{4'}$, die die vorstehend genannten Bedeutungen haben können,im Phenylkern substituiert ist, verethert und aus dem daraus resultierenden Aralkylether vom Typ 7

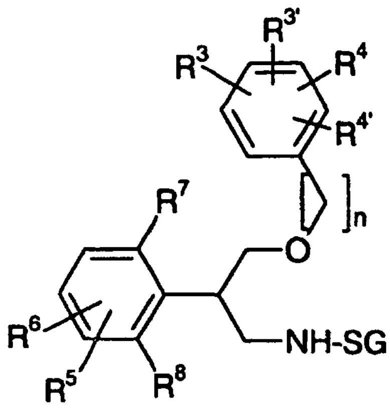

**7**

worin n die vorstehend genannten Bedeutungen haben kann, anschließend die Schutzgruppe aus dem geschützten Amin 7 abspaltet und das so erhaltene Amin der allgemeinen Formel 8

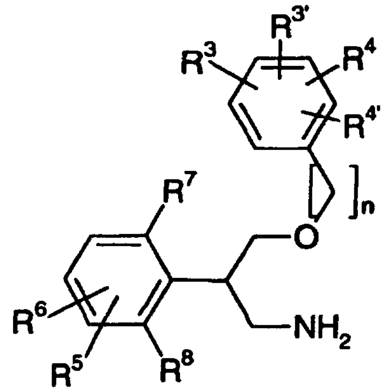

**8**

entweder durch Alkylierung mit einem Alkylierungsmittel vom Typ $R^1$-$Y^1$ und/oder $R^2$-$Y^2$, worin $R^1$ und $R^2$ die vorstehend genannten Bedeutungen haben können und in denen $Y^1$/$Y^2$ eine durch einen Aminostickstoff substituierbare Austrittsgruppe bedeutet oder durch Acylierung mit den entsprechenden Carbonsäurederivaten mit anschließender Reduktion und das so erhaltene Propylaminderivat der allgemeinen Formel 1 isoliert.

**11.** Verfahren zur Herstellung von Verbindungen der allgemeinen Formel 1, nach Anspruch 10, **dadurch gekennzeichnet, daß** man ein Benzylcyanid der allgemeinen Formel 2 mit einer metallorganischen Base in einem aprotischen Lösungsmittel bei einer Reaktionstemperatur in einem Intervall von -20 bis +30 °C deprotoniert, mit einem niederen Ameisensäurealkylester umsetzt, gegebenenfalls bei Raumtemperatur 25 °C nachreagieren läßt, die Reaktionsmischung hydrolysiert, das Reaktionsprodukt reinigt und isoliert und die so erhaltene Formylverbindung der allgemeinen Formel 3 mit einem Reduktionsmittel gegebenenfalls im Überschuß des Reduktionsmittels, der in einem Bereich von 5 bis 100 % liegt, in einem verzweigten oder unverzweigten Alkohol zu dem entsprechenden Alkohol reduziert und das Reaktionsprodukt 4 gegebenenfalls nach Zerstörung des überschüssigen Reduktionsmittel auf an sich aus dem Stand der Technik bekannte Weise isoliert und das so erhaltene Nitril 4 mit Wasserstoff in Gegenwart von Raney-Nickel in Gegenwart eines Amins in einem verzweigten oder unverzweigten Alkohol unter einem Wasserstoffdruck in einem Bereich von 10 bis 200 bar bei Reaktionstemperatur in einem Intervall von 20 bis 150 °C reduziert, das Reduktionsprodukt auf an sich bekannte Weise gegebenenfalls reinigt und isoliert und das so erhaltene Amin der allgemeinen Formel 5 entweder mit an sich bekannten Methoden in seine Enantiomeren der allgemeinen Formel 5a oder 5b auftrennt oder die Enantiomerenmischung des Amins der allgemeinen Formel 5 mit einem Reagenz vom Typ X-SG, worin X eine gegen einen Aminstickstoff substituierbare Austrittsgruppe und SG eine zum Schutz von primären Aminogruppen geeignete Schutzgruppe verkörpert selektiv schützt, wozu der racemische oder enantiomerenreine Aminoalkohol vom Typ 5 bzw. 5a oder 5b in einem unter den gegebenen Reaktionsbedingungen inerten Lösungsmittel löst, in einem Temperaturintervall von -20 bis 75 °C umsetzt, nach dem Abziehen des Lösungsmittels den Rückstand mit der wässerigen Lösung einer sauer reagierenden Verbindung und nach erfolgter Reaktion das geschützte Amin 6 isoliert, gegebenenfalls nach dessen Reinigung und das so erhaltene Reaktionsprodukt der allgemeinen Formel 6 in einem halogenierten niederen Kohlenwasserstoff löst und in Gegenwart eines Phasentransfer-Katalysators sowie in Gegenwart einer wässerigen Phase mit einem Phenylalkylderivat welches gegebenenfalls mit den Substituenten $R^3$, $R^3$, $R^4$ und/oder $R^4$ im Phenylkern substituiert ist, bei einer Temperatur in einem Intervall von +5 bis 60 °C durchführt, nach erfolgter Reaktion die organische Phase separiert und die wässerige Phase erschöpfend mit einem geeigneten Lösungsmittel extrahiert, die vereinigten organischen Phasen anschließend mit der wässerigen Lösung einer sauer reagierenden Verbindung wäscht, trocknet und eingeengt, den Rückstand nach an sich aus dem Stand der Technik bekannten Verfahren reinigt und das Reaktionsprodukt 7 isoliert, und anschließend die Schutzgruppe aus dem geschützten Amin 7 auf an sich aus dem Stand der Technik bekannte Art und Weise abspaltet und das so erhaltene Amin der allgemeinen Formel 8 mit den gewünschten Carbonsäurehalogeniden in einem inerten Lösungsmittel bei einer Reaktionstemperatur in einem Intervall von -50 bis 150 °C in Gegenwart eines geringen Überschusses an Acylierungsmittel acyliert und das daraus resultierende Amid vorzugsweise mit einem komplexen Hydridgegebenenfalls in Gegenwart eines Katalysators - in einem inerten Lösungsmittel bei einer Reaktionstemperatur in einem Intervall von 50 bis 150 °C umsetzt, das so erhaltene Propylaminderivat der allgemeinen Formel 1 nach an sich aus dem Stand der Technik bekannten Methoden reinigt und isoliert.

**12.** Verfahren zur Herstellung von Verbindungen der allgemeinen Formel 1,

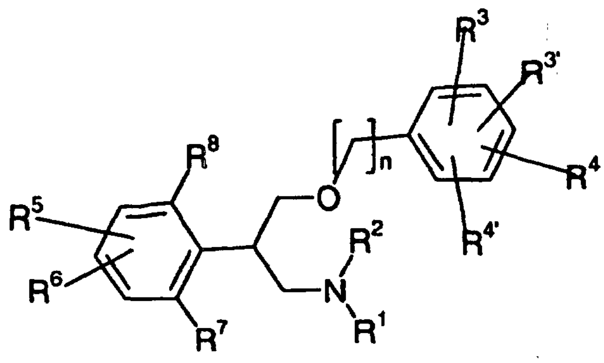

1

worin die Reste $R^1$, $R^2$, $R^3$, $R^{3'}$, $R^4$, $R^{4'}$, $R^5$, $R^6$, $R^7$, $R^8$ und n die in den Ansprüchen 1 bis 4 genannten Bedeutungen haben können,

**dadurch gekennzeichnet, daß** man ein geeignet substituiertes Amin $R^1R^2NH$, worin $R^1$ und $R^2$ die vorstehend genannten Bedeutungen haben kann, racemisch mit Epichlorhydrin bzw. stereospezifisch mit optisch aktivem Epichlorhydrin zum aminomethyl-substituierten Oxiran der allgemeinen Formel 9

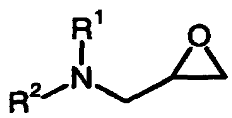

9

umsetzt und im anschließenden Reaktionsschritt mit einem Phenyl-Magnesiumhalogenid, wobei der Phenylring gegebenenfalls durch die Substituenten $R^5$, $R^6$, $R^7$ und/oder $R^8$, die die vorstehend genannten Bedeutungen haben können,substituiert sein kann, eine Grignard-Reaktion durchführt und den so erhaltenen Aminoalkohol der allgemeinen Formel **10a**

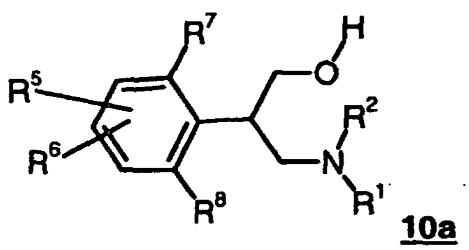

**10a**

mit Phenylalkylhalogeniden, bevorzugt Benzyhalogeniden, wobei der Phenylkern durch die Substituenten $R^3$ ,$R^3$, $R^4$ und/oder $R^{4'}$, die die vorstehend genannten Bedeutungen haben können, subtituiert sein kann, oder im Rahmen einer Mitsunobu-Reaktion oder Reppe Synthese zum Propylaminderivat der allgemeinen Formel 1 umsetzt und das Reaktionsprodukt nach an sich aus dem Stand der Technik bekannten Methoden reinigt und isoliert.

**Claims**

1. Compounds of general formula 1

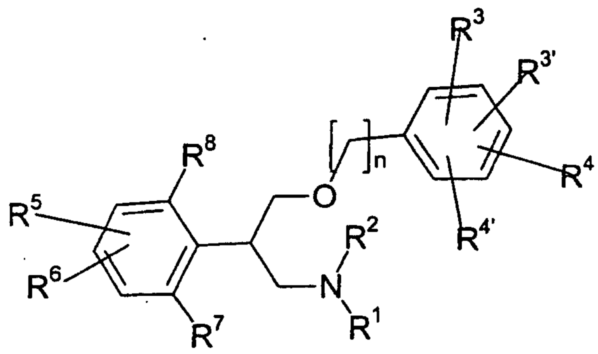

**1**

wherein

$R^1$ and $R^2$ independently of one another may denote hydrogen, $C_1$-$C_6$-alkyl, benzyl, furylmethyl, cycloalkyl, cycloalkyl-methyl, $C_2$-$C_6$-alkenyl, $C_2$-$C_6$-alkynyl, $C_1$-$C_6$-alkoxy-$(CH_2)_l$-, $C_{3}$-$_{8}$-cycloalkoxy-$(CH_2)_m$- and
1 denotes an integer 1, 2, 3 or 4,
and
m denotes an integer 0, 1, 2, 3 or 4,
or

$R^1$ and $R^2$ together with the nitrogen atom form a 5-, 6-, or 7-membered heterocyclic ring, which may optionally be substituted with 1-4 methyl groups or a dimethylene group;

n may denote an integer 0, 1, 2 or 3;

$R^3$, $R^4$ and $R^{3'}$ independently of one another may denote hydrogen, fluorine, chlorine, bromine, hydroxy, methyl, ethyl, methoxy or $CF_3$;

$R^{4'}$ may denote hydrogen;

$R^5$ and $R^6$ independently of one another may denote hydrogen, fluorine, chlorine, bromine, methyl or ethyl or

$R^5$ and $R^6$ adjacent to one another denote a fused-on aromatic ring;

$R^7$ and $R^8$ independently of one another may denote hydrogen, methyl, ethyl, methoxy or fluorine,

optionally in the form of the racemates, the enantiomers, the diastereomers, tautomers and mixtures thereof and optionally the pharmacologically acceptable addition salts thereof.

**2.** Compounds of general formula **1** according to claim 1, wherein:

$R^1$ and $R^2$ independently of one another may denote hydrogen, $C_1$-$C_6$-alkyl, benzyl, furylmethyl, cycloalkyl, cycloalkyl-methyl, $C_2$-$C_6$-alkenyl, $C_{2-6}$-alkynyl, $C_{1-6}$-alkoxy-$(CH_2)_1$-, $C_3$-$C_8$-cycloalkoxy-$(CH_2)_m$- , and
1 denotes an integer 1, 2, 3 or 4,
and
m denotes an integer 0, 1, 2, 3 or 4,
or

$R^1$ and $R^2$ together with the nitrogen atom form a 5-, 6-, or 7-membered heterocyclic ring, which may optionally be substituted with 1-4 methyl groups or a dimethylene group;

n may denote an integer 0, 1, 2 or 3;

$R^3$ and $R^4$ independently of one another may denote hydrogen, fluorine, chlorine, bromine, hydroxy, methyl, ethyl, methoxy or $CF_3$;

$R^{3'}$ and $R^{4'}$ may denote hydrogen;

$R^5$ and $R^6$ independently of one another may denote hydrogen, fluorine, chlorine, bromine, methyl or ethyl;

$R^7$ and $R^8$ independently of one another may denote hydrogen, methyl, ethyl, methoxy or fluorine,

optionally in the form of the racemates, the enantiomers, the diastereomers, tautomers and mixtures thereof and optionally the pharmacologically acceptable addition salts thereof.

**3.** Compounds of general formula **1** according to one of claims 1 or 2, wherein

$R^1$ and $R^2$ independently of one another denote hydrogen, $C_{1}$-$_{4}$-alkyl, benzyl, furylmethyl, cycloalkyl, cycloalkyl-methyl, $C_2$ -$_4$-alkenyl, $C_2$-$C_4$-alkynyl, $C_1$-$C_4$-alkoxy-$(CH_2)_l$-, $C_3$-$C_6$-cycloalkoxy-$(CH_2)_m$-
and
1 denotes an integer 1, 2 or 3,
and
m denotes an integer 1, 2 or 3,
or

$R^1$ and $R^2$ together with the nitrogen atom form a 5- or 6- membered heterocyclic ring which may optionally be substituted with 1, 2 or 3 methyl groups or a dimethylene group;

n may denote an integer 0, 1, 2 or 3;

$R^3$ may denote fluorine, chlorine or methyl;

$R^4$ may denote hydrogen, fluorine, chlorine or methyl;

$R^{3'}$ and $R^{4'}$ may denote hydrogen;

$R^5$ and $R^6$ independently of one another may denote hydrogen or methyl;

$R^7$ and $R^8$ independently of one another may denote methyl, ethyl or methoxy,

optionally in the form of the racemates, the enantiomers, the diastereomers, tautomers and mixtures thereof and optionally the pharmacologically acceptable addition salts thereof.

**4.** Compounds of general formula **1** according to one of claims 1 to 3, wherein

$R^1$ and $R^2$ independently of one another denote hydrogen, methyl, ethyl, propyl, butyl, benzyl, furylmethyl, cyclopentyl, cyclohexyl, cyclopropylmethyl, cyclobutylmethyl, cyclopentylmethyl, cyclohexylmethyl, $C_2$-$C_4$-alkenyl, preferably allyl, $C_2$-$C_4$-alkynyl, preferably propargyl, $C_1$-$C_4$-alkoxy-$(CH_2)_1$-, $C_3$-$C_6$-cycloalkoxy- $(CH_2)_m$-,
and
1 denotes an integer 1, 2 or 3
and
m denotes an integer 1, 2 or 3,
or

$R^1$ and $R^2$ together with the.nitrogen atom form a 5- or 6- membered heterocyclic ring which may optionally be substituted with 1 or 2 methyl groups or a dimethylene group;

n may denote 1;

$R^3$ may denote ortho-fluorine, ortho-chlorine or ortho-methyl;

| | |
|---|---|
| $R^4$ | may denote hydrogen, *ortho*-fluorine, *ortho*-chlorine or *ortho*-methyl; |
| $R^{3'}$ and $R^{4'}$ | may denote hydrogen; |
| $R^5$ and $R^6$ | may denote hydrogen; |
| $R^7$ and $R^8$, | which may be identical or different, may denote methyl or ethyl, |

optionally in the form of the racemates, the enantiomers, the diastereomers, tautomers and mixtures thereof and optionally the pharmacologically acceptable addition salts thereof.

**5.** Pharmaceutical preparation, **characterised in that** it contains one of the compounds according to one of claims 1 to 4 and the acid addition salts thereof together with conventional excipients and carriers.

**6.** Pharmaceutical preparation according to claim 5, **characterised in that** it is formulated as a solution for infusion.

**7.** Use of compounds according to one of claims 1 to 4 for preparing a pharmaceutical composition for the therapeutic treatment of functional disorders caused by overstimulation.

**8.** Use of compounds according to one of claims 1 to 4 for preparing a pharmaceutical composition for the therapeutic treatment of arrhythmias, spasms, cardiac and cerebral ischaemias, pain and neurodegenerative disorders of various origins.

**9.** Use according to claim 7 or 8 for preparing a pharmaceutical composition for the therapeutic treatment of epilepsy, hypoglycaemia, hypoxia, anoxia, brain trauma, brain oedema, cerebral stroke, perinatal asphyxia, degeneration of the cerebellum, amyotropic lateral sclerosis, Huntington's disease, Alzheimer's disease, Parkinson's disease, cyclophrenia, hypotonia, cardiac infarct, heart rhythm disorders, angina pectoris, chronic pain, neuropathic pain and local anaesthesia.

**10.** Process for preparing compounds of general formula 1,

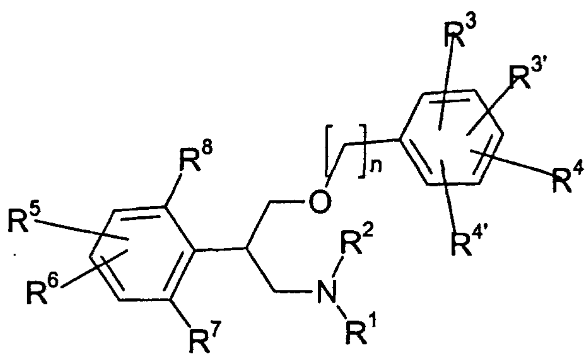

**1**

wherein the groups $R^1$, $R^2$, $R^3$, $R^{3'}$, $R^4$, $R^{4'}$, $R^5$, $R^6$, $R^7$, $R^8$ and n may have the meanings given in claims 1 to 4, **characterised in that** a benzylcyanide of general formula **2**

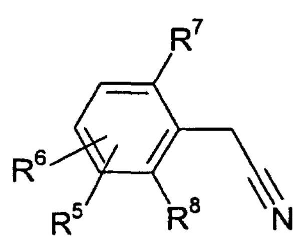

**2**

wherein the groups $R^5$ to $R^7$ may have the meanings given hereinbefore, is deprotonated, reacted with a formylating agent, the resulting formyl compound of general formula 3

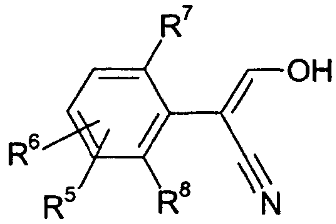

3

and the corresponding alcohol are reduced and the reaction product **4**

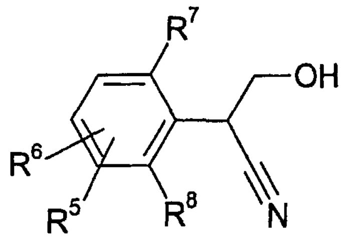

4

is reduced to the corresponding amine of general formula 5

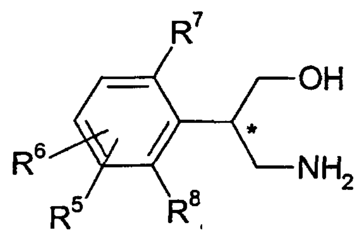

5

this is optionally separated, by methods known *per se* from the prior art, with malic acid, tartaric acid, mandelic acid or camphorsulphonic acid, of which tartaric acid is particularly preferred, into its enantiomers of general formula 5a or 5b

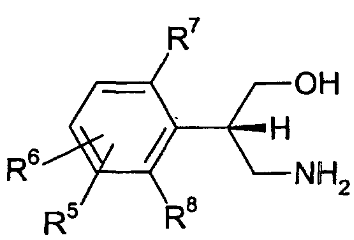

5a

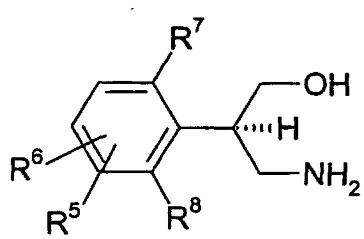

5b

or the mixture of enantiomers of the amine of general formula **5** is converted with a suitable reagent of type X-SG (wherein X denotes a leaving group which may be substituted by an amino nitrogen and SG denotes a protecting group suitable for a primary amine) into the protected amine of general formula 6

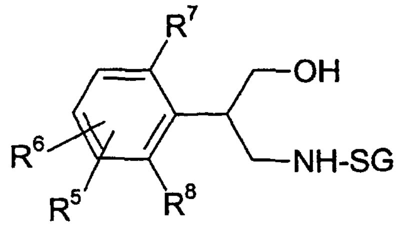

6

and the amine thus protected is etherified with a suitable phenylalkyl derivative which is optionally substituted by the substituents $R^3$, $R^{3'}$, $R^4$ and $R^{4'}$ in the phenyl nucleus and from the resulting aralkylether of type 7

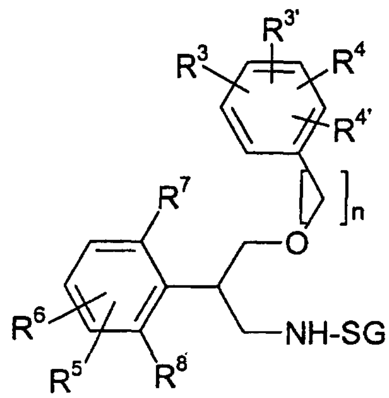

7

wherein n may have the meanings given hereinbefore, then the protecting group is cleaved from the protected amine 7 and the resulting amine of general formula 8

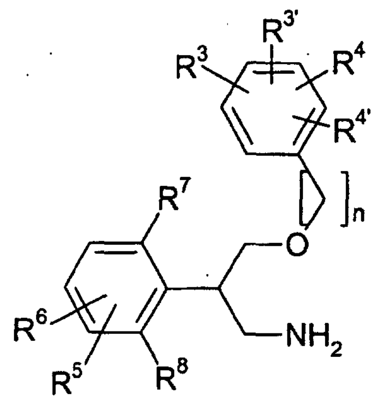

either by alkylation with an alkylating agent of the type $R^1$-$Y^1$ and/or $R^2$-$Y^2$, wherein $R^1$ and $R^2$ may have the meanings given hereinbefore and wherein $Y^1$/$Y^2$ denotes a leaving group which may be substituted by an amino nitrogen or by acylation with the corresponding carboxylic acid derivatives with subsequent reduction and the resulting propylamine derivative of general formula 1 is isolated.

**11.** Process for preparing compounds of general formula 1, according to claim 10, **characterised in that** a benzylcyanide of general formula 2 is deprotonated with an organometallic base in an aprotic solvent at a reaction temperature in the range from -20 to +30°C , reacted with a lower alkyl formate, optionally left to continue reacting at ambient temperature 25°C, the reaction mixture is hydrolysed, the reaction product is purified and isolated and the resulting formyl compound of general formula 3 is reduced with a reducing agent, optionally in an excess of

the reducing.agent, which is in the range from 5 to 100 % and preferably in the range from 50 to 100 % and most preferably in the range from 70 to 90 %, in a branched or unbranched alcohol to obtain the corresponding alcohol and the reaction product **4** is isolated, optionally after destruction of the excess reducing agent, by a method known *per se* from the prior art and the resulting nitrile **4** is reduced with hydrogen in the presence of Raney nickel in the presence of an amine in a branched or unbranched alcohol under a hydrogen pressure in the range from 10 to 200 bar, at a reaction temperature in the range from 20 to 150°C, the reduction product is optionally purified and isolated in a manner known per se and the resulting amine of general formula **5** is either separated by methods known *per se* into its enantiomers of general formula **5a** or **5b** or the mixture of enantiomers of the amine of general formula **5** is selectively protected with a reagent of type X-SG, wherein X denotes a leaving group which can be substituted by an amino nitrogen and SG denotes a protecting group suitable for protecting primary amino groups, for which purpose the racemic or enantiomerically pure aminoalcohol of type **5** or **5a** or **5b** is dissolved in a solvent which is inert under the particular reaction conditions, is reacted in the temperature range from -20 to 75°C, after the elimination of the solvent the residue with the aqueous solution of an acidically reacting compound, and after the reaction has ended the protected amine 6 is isolated, optionally after the purification thereof, and the resulting reaction product of general formula 6 is dissolved in a halogenated lower hydrocarbon, and is carried out in the presence of a phase transfer catalyst and in the presence of an aqueous phase with a phenylalkyl derivative which is optionally substituted by the substituents $R^3$, $R^{3'}$, $R^4$ and/or $R^{4'}$ in the phenyl nucleus, at a temperature in the range from +5 to 60°C, after the reaction has ended the organic phase is separated and the aqueous phase is exhaustively extracted with a suitable solvent, the combined organic phases are then washed with the aqueous solution of an acidically reacting compound, then dried and concentrated by evaporation, the residue is purified by methods known *per se* from the prior art and the reaction product **7** is isolated, and then the protecting group is cleaved from the protected amine **7** in a manner known *per* se from the prior art and the resulting amine of general formula **8** is acylated with the desired carboxylic acid halides in an inert solvent at a reaction temperature in the range from - 50 to 150°C in the presence of a slight excess of acylating agent and the resulting amide is preferably reacted with a complex hydride, optionally in the presence of a catalyst - in an inert solvent, at a reaction temperature in the range from 50 to 150°C, the resulting propylamine derivative of general formula **1** is purified using methods known *per se* from the prior art and isolated.

**12.** Process for preparing compounds of general formula **1**,

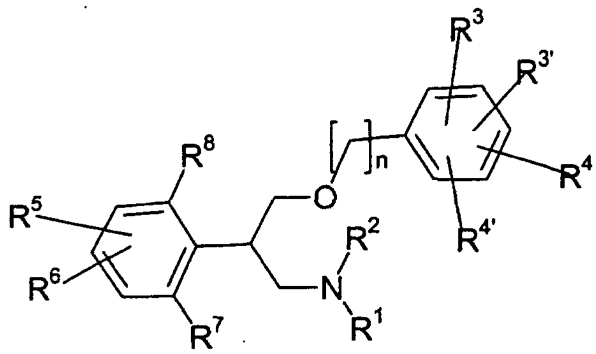

**1**

wherein the groups $R^1$, $R^2$, $R^3$, $R^{3'}$, $R^4$, $R^{4'}$, $R^5$, $R^6$, $R^7$, $R^8$ and n may have the meanings given in claims 1 to 4, **characterised in that** a suitably substituted amine $R^1R^2NH$ wherein the groups $R^1$ and $R^2$ may have the meanings given hereinbefore, is reacted racemically with epichlorohydrin or stereospecifically with optically active epihlorohydrin to yield the aminomethyl-substituted oxirane of general formula **9**

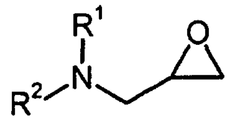

**9**

and in the subsequent reaction step a Grignard reaction is carried out with a phenyl-magnesium halide, wherein the phenyl ring may optionally be substituted by the substituents $R^5$, $R^6$, $R^7$ and/or $R^8$, which may have the meanings given hereinbefore, and the resulting aminoalcohol of general formula **10a**

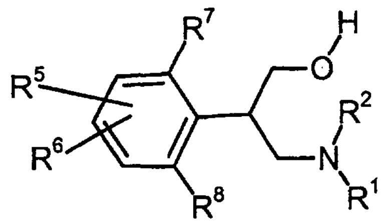

**10a**

is reacted with phenylalkyl halides, preferably benzyl halides, whilst the phenyl nucleus may be substituted by the substituents $R^3$, $R^{3'}$, R4 and/or $R^{4'}$, which may have the meanings given hereinbefore, or is reacted within the scope of a Mitsunobu reaction or Reppe synthesis to yield the propylamine derivative of general formula **1** and the reaction product is purified by methods known per se from the prior art and isolated.

## Revendications

**1.** Composés de formule générale 1

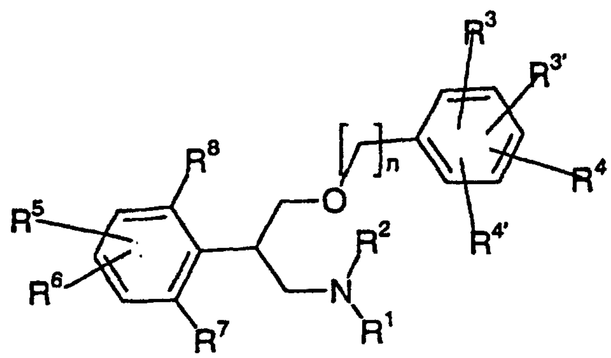

**1**

où

$R^1$ et $R^2$ peuvent représenter indépendamment l'un de l'autre l'hydrogène, alkyle en $C_1$-$C_6$, benzyle, furylméthyle, cycloalkyle, cycloalkyl-méthyle, alcényle en $C_2$-$C_6$, alcynyle en $C_2$-$C_6$, alcoxy en $C_1$-$C_6$-$(CH_2)_1$-, cycloalcoxy en $C_3$-$C_8$-$(CH_2)_m$-,

et

1 peut représenter un nombre entier 1, 2, 3 ou 4,

et

m peut représenter un nombre entier 0, 1, 2, 3 ou 4,

ou

$R^1$ et $R^2$ peuvent former avec l'atome d'azote un cycle hétérocyclique à 5, 6 ou 7 chaînons qui peut éventuellement être substitué par 1-4 groupes méthyle

ou un groupe diméthylène ;

n peut représenter un nombre entier 0, 1, 2 ou 3 ;

$R^3$, $R^4$ et $R^{3'}$ peuvent représenter indépendamment les uns des autres l'hydrogène, le fluor, le chlore, le brome, hydroxyle, méthyle, éthyle, méthoxy ou $CF_3$ ;

$R^{4'}$ peut représenter l'hydrogène ;

$R^5$ et $R^6$ peuvent représenter indépendamment l'un de l'autre l'hydrogène, le fluor, le chlore, le brome, méthyle ou éthyle ou

$R^5$ et $R^6$ voisins peuvent représenter un cycle aromatique condensé ;

$R^7$ et $R^8$ peuvent représenter indépendamment l'un de l'autre méthyle, éthyle, méthoxy ou le fluor,

éventuellement sous forme de leurs racémates, de leurs énantiomères, de leurs diastéréoisomères, de leurs tautomères et de leurs mélanges, et éventuellement de leurs sels d'addition d'acide pharmacologiquement irréprochables.

**2.** Composés de formule générale 1 selon la revendication 1 où
$R^1$ et $R^2$ peuvent représenter indépendamment l'un de l'autre l'hydrogène, alkyle en $C_1$-$C_6$, benzyle, furylméthyle, cycloalkyle, cycloalkyl-méthyle, alcényle en $C_2$-$C_6$, alcynyle en $C_2$-$C_6$, alcoxy en $C_1$-$C_6$-$(CH_2)_1$-, cycloalcoxy en $C_3$-$C_8$-$(CH_2)_m$-,
et
1 peut représenter un nombre entier 1, 2, 3 ou 4,
et
m peut représenter un nombre entier 0, 1, 2, 3 ou 4,
ou
$R^1$ et $R^2$ peuvent former avec l'atome d'azote un cycle hétérocyclique à 5, 6 ou 7 chaînons qui peut éventuellement être substitué par 1-4 groupes méthyle
ou un groupe diméthylène ;
n peut représenter un nombre entier 0, 1, 2 ou 3 ;
$R^3$ et $R^4$ peuvent représenter indépendamment l'un de l'autre l'hydrogène, le fluor, le chlore, le brome, hydroxyle, méthyle, éthyle, méthoxy ou $CF_3$ ;
$R^{3'}$ et $R^{4'}$ peut représenter l'hydrogène ;
$R^5$ et $R^6$ peuvent représenter indépendamment l'un de l'autre l'hydrogène, le fluor, le chlore, le brome, méthyle ou éthyle ;
$R^7$ et $R^8$ peuvent représenter indépendamment l'un de l'autre méthyle, éthyle, méthoxy ou le fluor,
éventuellement sous forme de leurs racémates, de leurs énantiomères, de leurs diastéréoisomères, de leurs tautomères et de leurs mélanges, et éventuellement de leurs sels d'addition d'acide pharmacologiquement irréprochables.

**3.** Composés de formule générale 1 selon l'une des revendications 1 ou 2 où
$R^1$ et $R^2$ peuvent représenter indépendamment l'un de l'autre l'hydrogène, alkyle en $C_1$-$C_4$, benzyle, furylméthyle, cycloalkyle, cycloalkyl-méthyle, alcényle en $C_2$-$C_4$, alcynyle en $C_2$-$C_4$, alcoxy en $C_1$-$C_4$-$(CH_2)_1$-, cycloalcoxy en $C_3$-$C_6$-$(CH_2)_m$-,
et
1 peut représenter un nombre entier 1, 2: ou 3,
et
m peut représenter un nombre entier 1, 2 ou 3,
ou
$R^1$ et $R^2$ peuvent former avec l'atome d'azote un cycle hétérocyclique à 5 ou 6 chaînons qui peut éventuellement être substitué par 1, 2 ou 3 groupes méthyle ou un groupe diméthylène ;
n peut représenter un nombre entier 0, 1, 2 ou 3 ;
$R^3$ peut représenter le fluor, le chlore ou méthyle ;
$R^4$ peut représenter l'hydrogène, le fluor, le chlore ou méthyle ;
$R^{3'}$ et $R^{4'}$ peuvent représenter l'hydrogène ;
$R^5$ et $R^6$ peuvent représenter indépendamment l'un de l'autre l'hydrogène ou méthyle ;
$R^7$ et $R^8$ peuvent représenter indépendamment l'un de l'autre méthyle, éthyle ou méthoxy,
éventuellement sous forme de leurs racémates, de leurs énantiomères, de leurs diastéréoisomères, de leurs tautomères et de leurs mélanges, et éventuellement de leurs sels d'addition d'acide pharmacologiquement irréprochables.

**4.** Composés de formule générale 1 selon l'une des revendications 1 à 3 où
$R^1$ et $R^2$ peuvent représenter indépendamment l'un de l'autre l'hydrogène, méthyle, éthyle, propyle, butyle, benzyle, furylméthyle, cyclopentyle, cyclohexyle, cyclopropylméthyle, cyclobutylméthyle, cyclopentylméthyle, cyclohexylméthyle, alcényle en $C_2$-$C_4$, de préférence allyle, alcynyle en $C_2$-$C_4$, de préférence propargyle, alcoxy en $C_1$-$C_4$-$(CH_2)_1$-, cycloalcoxy en $C_3$-$C_6$-$(CH_2)_m$-,
et
1 peut représenter un nombre entier 1, 2 ou 3,
et
m peut représenter un nombre entier 1, 2 ou 3,
ou
$R^1$ et $R^2$ peuvent former avec l'atome d'azote un cycle hétérocyclique à 5 ou 6 chaînons qui peut éventuellement être substitué par 1 ou 2 groupes méthyle ou un groupe diméthylène ;
n peut représenter 1 ;
$R^3$ peut représenter *ortho*-fluoro, *ortho*-chloro ou *ortho*-méthyle ;

$R^4$ peut représenter l'hydrogène, *ortho*-fluoro, *ortho*-chloro ou *ortho*-méthyle ;
$R^{3'}$ et $R^{4'}$ peuvent représenter l'hydrogène ;
$R^5$ et $R^6$ peuvent représenter l'hydrogène ;
$R^7$ et $R^8$, identiques ou différents, peuvent représenter méthyle ou éthyle,
éventuellement sous forme de leurs racémates, de leurs énantiomères, de leurs diastéréoisomères, de leurs tautomères et de leurs mélanges, et éventuellement de leurs sels d'addition d'acide pharmacologiquement irréprochables.

**5.** Préparation pharmaceutique **caractérisée par** une teneur en l'un des composés selon l'une des revendications 1 à 4 et ses sels d'addition d'acide, outre des adjuvants et supports courants.

**6.** Préparation pharmaceutique selon la revendication 5 **caractérisée en ce qu'**elle est formulée sous forme de solution pour perfusion.

**7.** Utilisation de composés selon l'une des revendications 1 à 4 pour la production d'un médicament pour le traitement thérapeutique de troubles fonctionnels dus à une surexcitation.

**8.** Utilisation de composés selon l'une des revendications 1 à 4 pour la production d'un médicament pour le traitement thérapeutique d'arythmies, de spasmes, d'ischémies cardiaques et cérébrales, de douleurs et de maladies neurodégénératives de genèse diverse.

**9.** Utilisation selon la revendication 7 ou 8 pour la production d'un médicament pour le traitement thérapeutique de l'épilepsie, de l'hypoglycémie, de l'hypoxie, de l'anoxie, des traumatismes cérébraux, de l'oedème cérébral, de l'apoplexie cérébrale, de l'asphyxie périnatale, des dégénérescences du cervelet, de la sclérose latérale amyotrophique, de la maladie de Huntington, de la maladie d'Alzheimer, de la maladie de Parkinson, de la cyclothymie, de l'hypotonie, de l'infarctus cardiaque, des troubles du rythme cardiaque, de l'angine de poitrine, de la douleur chronique, de la douleur névropathique et de l'anesthésie locale.

**10.** Procédé de production de composés de formule générale 1

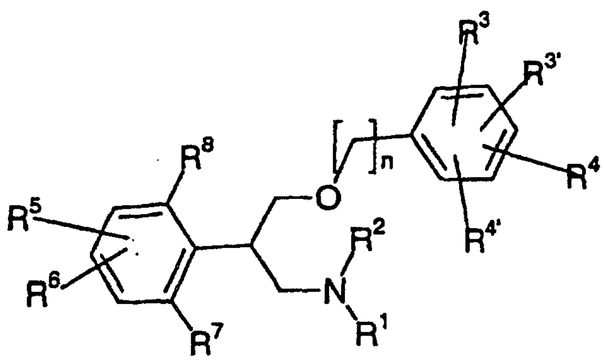

**1**

où les restes $R^1$, $R^2$, $R^3$, $R^{3'}$, $R^4$, $R^{4'}$, $R^5$, $R^6$, $R^7$, $R^8$ et n peuvent avoir les significations citées dans les revendications 1 à 4,
**caractérisé en ce que** l'on déprotone un cyanure de benzyle de formule générale 2

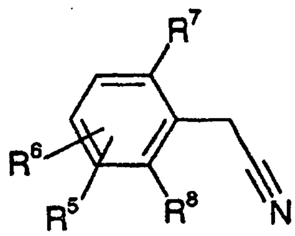

**2**

où les restes $R^5$ à $R^7$ peuvent avoir les significations citées précédemment, on le fait réagir avec un agent de formylation, on réduit le composé formyle de formule générale 3 ainsi obtenu

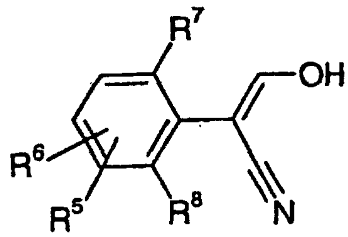

**3**

et l'alcool correspondant, et on réduit le produit réactionnel **4**

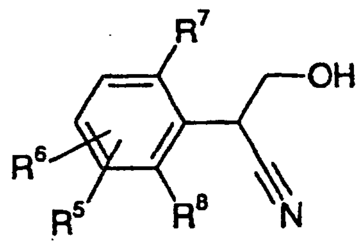

**4**

en l'amine de formule générale 5 correspondante

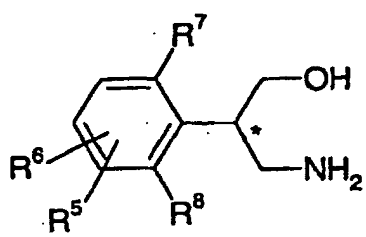

**5**

on résout celle-ci, éventuellement avec des méthodes connues en soi par l'état de la technique avec l'acide malique, l'acide tartrique, l'acide mandélique ou l'acide camphosulfonique, parmi lesquels l'acide tartrique est particulièrement préféré, en ses énantiomères de formule générale 5a ou 5b

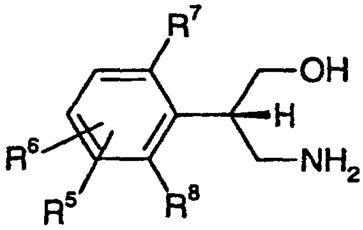

5a

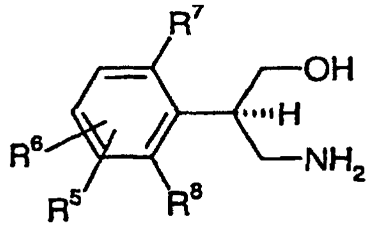

5b

ou on convertit le mélange d'énantiomères de l'amine de formule générale 5 avec un réactif approprié de type X-SG où X représente un groupe partant substituable par un azote d'amine et SG représente un groupe protecteur approprié pour une amine primaire, en l'amine protégée de formule générale 6

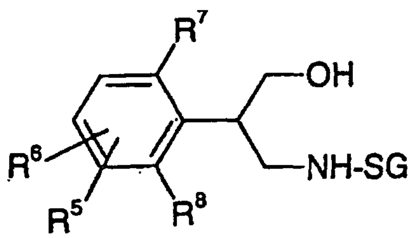

6

et on éthérifie l'amine ainsi protégée avec un dérivé phénylalkyle approprié qui est éventuellement substitué sur le noyau phényle par les substituants $R^3$, $R^{3'}$, $R^4$ et $R^{4'}$ qui peuvent avoir les significations citée précédemment, puis, de l'aralkyléther de type 7 résultant

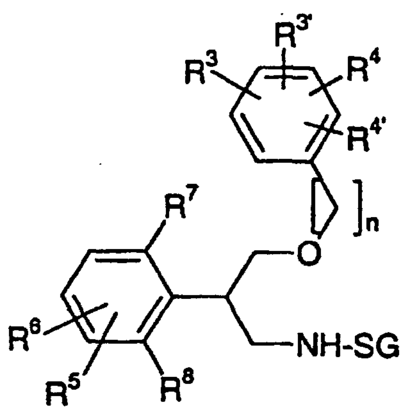

7

où n peut avoir les significations citées précédemment, on clive le groupe protecteur de l'amine protégée 7 et on fait réagir l'amine de formule générale 8 ainsi obtenue

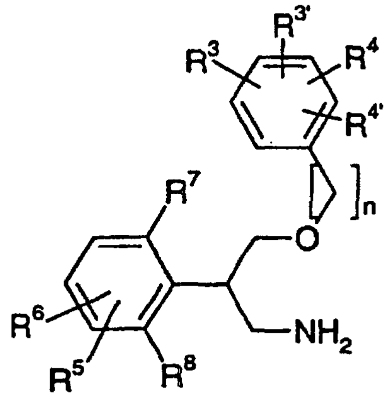

**8**

par alkylation avec un agent d'alkylation de type $R^1$-$Y^1$ et/ou $R^2$-$Y^2$ où $R^1$ et $R^2$ peuvent avoir les significations citées précédemment et où $Y^1$/$Y^2$ représenter un groupe partant substituable par un azote d'amine ou par acylation avec les dérivés d'acides carboxyliques correspondants, avec réduction subséquente, et on isole le dérivé de propylamine de formule générale 1 ainsi obtenu.

**11.** Procédé de production de composés de formule générale 1 selon la revendication 10 **caractérisé en ce que** l'on déprotone un cyanure de benzyle de formule générale 2 avec une base organométallique dans un solvant aprotique à une température de réaction dans un intervalle de -20 à +30°C, on fait réagir avec un formiate d'alkyle inférieur, on laisse éventuellement réagir encore à température ambiante de 25°C, on hydrolyse le mélange réactionnel, on purifie et isole le produit réactionnel et on réduit en l'alcool correspondant le composé formyle de formule générale 3 ainsi obtenu avec un réducteur, éventuellement dans un excès du réducteur qui est situé dans un domaine de 5 à 100 %, dans un alcool ramifié ou non ramifié, et on isole le produit réactionnel 4 éventuellement après destruction du réducteur en excès de manière connue en soi par l'état de la technique et on réduit le nitrile 4 ainsi obtenu avec l'hydrogène en présence de nickel de Raney en présence d'une amine dans un alcool ramifié ou non ramifié sous une pression d'hydrogène dans un domaine de 10 à 200 bar à une température de réaction dans un intervalle de 20 à 150°C, on purifie éventuellement et isole le produit réactionnel de manière connue en soi et on résout l'amine de formule générale 5 ainsi obtenue avec des méthodes connues en soi en ses énantiomères de formule générale 5a ou 5b ou on protège sélectivement le mélange d'énantiomères de l'amine de formule générale 5 avec un réactif de type X-SG où X représente un groupe partant substituable par un azote d'amine et SG représente un groupe protecteur approprié pour la protection de groupes amino primaires, pour ce faire on dissout l'aminoalcool racémique ou énantiomériquement pur de type 5 ou 5a ou 5b dans un solvant inerte dans les conditions réactionnelles données, on le fait réagir dans un intervalle de température de -20 à 75°C, après élimination du solvant on fait réagir le résidu avec la solution aqueuse d'un composé à réaction acide et après la fin de la réaction on isole l'amine protégée 6, éventuellement après sa purification, et on dissout le produit réactionnel de formule générale 6 ainsi obtenu dans un hydrocarbure inférieur halogéné et on le fait réagir en présence d'un catalyseur de transfert de phase ainsi qu'en présence d'une phase aqueuse avec un dérivé phénylalkyle qui est éventuellement substitué sur le noyau phényle par les substituants $R^3$, $R^{3'}$, $R^4$ et/ou $R^{4'}$, à une température dans un intervalle de +5 à 60°C, après la fin de la réaction on sépare la phase organique et on extrait totalement la phase aqueuse avec un solvant approprié, puis on lave les phases organiques réunies avec la solution aqueuse d'un composé à réaction acide, on sèche et on concentre, on purifie le résidu selon des procédés connus en soi par l'état de la technique et on isole le produit réactionnel 7, puis on clive le groupe protecteur de l'amine protégée 7 de manière connue en soi par l'état de la technique et on acyle l'amine de formule générale 8 ainsi obtenue avec les halogénures d'acides carboxyliques souhaités dans un solvant inerte à une température de réaction dans un intervalle de -50 à 150°C en présence d'un faible excès d'agent d'acylation et on fait réagir l'amide résultant de préférence avec un hydrure complexe, éventuellement en présence d'un catalyseur, dans un solvant inerte à une température de réaction dans un intervalle de 50 à 150°C, on purifie et isole le dérivé de propylamine de formule générale 1 selon des méthodes connues en soi par l'état de la technique.

**12.** Procédé de production de composés de formule générale 1

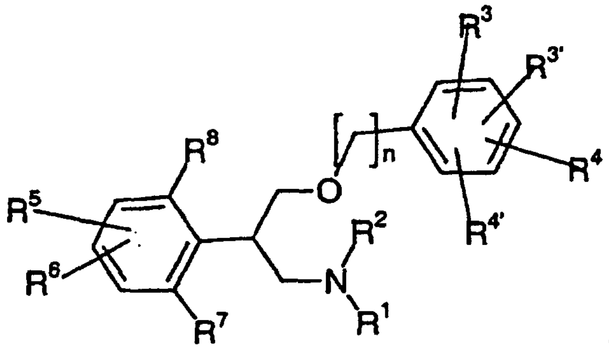

1

où les restes $R^1$, $R^2$, $R^3$, $R^{3'}$, $R^4$, $R^{4'}$, $R^5$, $R^6$, $R^7$, $R^8$ et n peuvent avoir les significations citées dans les revendications 1 à 4,
**caractérisé en ce que** l'on convertit une amine substituée de manière appropriée $R^1R^2NH$ où $R^1$ et $R^2$ peuvent avoir les significations citées précédemment, de manière racémique avec l'épichlorhydrine ou de manière stéréospécifique avec une épichlorhydrine optiquement active en l'oxirane aminométhyl-substitué de formule générale 9

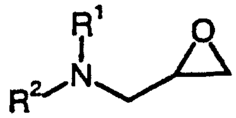

9

et dans l'étape réactionnelle suivante on conduit une réaction de Grignard avec un halogénure de phénylmagnésium où le cycle phényle peut éventuellement être substitué par les substituants $R^5$, $R^6$, $R^7$ et/ou $R^8$ qui peuvent avoir les significations citées précédemment, et on convertit l'aminoalcool de formule générale 10a ainsi obtenu

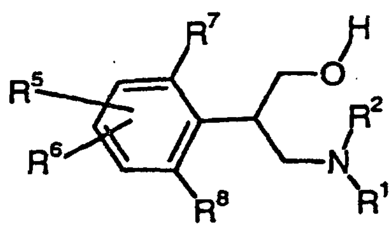

10a

avec des halogénures de phénylalkyle, de préférence des halogénures de benzyle, où le noyau phényle peut être substitué par les substituants $R^3$, $R^{3'}$ , $R^4$ et/ou $R^{4'}$, qui peuvent avoir les significations citées précédemment, ou dans le cadre d'une réaction de Mitsunobu ou d'une synthèse de Repp en le dérivé de propylamine de formule générale 1 et on purifie et isole le produit réactionnel selon des méthodes connues en soi par l'état de la technique.